## Europäisches Patentamt
## European Patent Office
## Office européen des brevets

(19)

(11) Veröffentlichungsnummer : **0 430 929 B1**

(12) **EUROPÄISCHE PATENTSCHRIFT**

(45) Veröffentlichungstag der Patentschrift :
**01.06.94 Patentblatt 94/22**

(51) Int. Cl.$^5$ : **A61B 17/39**

(21) Anmeldenummer : **91103094.8**

(22) Anmeldetag : **17.07.86**

(54) **Hochfrequenz-Chirurgiegerät für die thermische Koagulation biologischer Gewebe.**

(43) Veröffentlichungstag der Anmeldung :
**05.06.91 Patentblatt 91/23**

(60) Veröffentlichungsnummer der früheren
Anmeldung nach Art. 76 EPü : **0 253 012**

(45) Bekanntmachung des Hinweises auf die
Patenterteilung :
**01.06.94 Patentblatt 94/22**

(84) Benannte Vertragsstaaten :
**DE FR GB IT**

(56) Entgegenhaltungen :
**EP-A- 0 219 568**
**DE-A- 3 120 102**
**FR-A- 1 347 865**
**FR-A- 2 474 307**

(73) Patentinhaber : **Erbe Elektromedizin GmbH.**
**Waldhörnlestrasse 17**
**D-72072 Tübingen (DE)**

(72) Erfinder : **Farin, Günter**
**Kapellenweg 15**
**W-7400 Tübingen-Hirschau (DE)**
Erfinder : **Haag, Reiner**
**Bulzinger Strasse 59**
**W-7201 Rietheim (DE)**
Erfinder : **Pütz, Peter**
**Weissdornweg 14/116**
**W-7400 Tübingen (DE)**

(74) Vertreter : **Endlich, Fritz, Dipl.-Phys. et al**
**Patentanwalt**
**Postfach 13 26**
**D-82101 Germering (DE)**

## Beschreibung

Die Erfindung betrifft ein Hochfrequenz-Chirurgiegerät für die thermische Koagulation biologischer Gewebe mittels hochfrequenten elektrischen Wechselstromes entsprechend dem Oberbegriff des Patentanspruchs 1, welche den Koagulationsvorgang automatisch beendet, sobald der Koagulationsvorgang ein bestimmtes Stadium erreicht hat.

Die thermische Koagulation biologischer Gewebe, im folgenden kurz Koagulation genannt, mittels hochfreqenten elektrischen Wechselstromes, im folgenden kurz HF-Strom genannt, wird seit über 50 Jahren in der Human- und Veterinärmedizin zum Devitalisieren kranker Gewebe und zum Verschließen perforierter Blutgefäße angewendet. Hierzu wird der HF-Strom durch das zu koagulierende Gewebe geleitet, so daß die Erwärmung des durchflossenen Gewebes endogen erfolgt. Hierbei ist die Erwärmung des Gewebes von verschiedenen Parametern abhängig wie beispielsweise der Menge des zu koagulierenden Gewebes, der Intensität des HF-Stromes, dem spezifischen elektrischen Widerstand des zu koagulierenden Gewebes, der Stromflußdauer des HF-Stromes, der spezifischen Wärmekapazität des zu koagulierenden Gewebes, der Form und Größe der Koagulationselektroden, der Wärmeableitung durch die Koagulationselektroden, auch davon, ob die Koagulation monopolar oder bipolar ausgeführt wird.

Da diese Parameter sowohl während eines Koagulationsvorganges als auch von Koagulationsvorgang zu Koagulationsvorgang mehr oder weniger stark variieren, ist die Reproduzierbarkeit einer gewünschten Koagulationsqualität sehr schwierig zu realisieren und erfordert große Aufmerksamkeit und Erfahrung des Operateurs.

In den verschiedenen chirurgischen Fachbereichen haben sich unterschiedliche Koagulationstechniken herausgebildet, weiche adäquate Eigenschaften der verwendeten Einrichtungen für die Koagulation erfordern. In der Regel werden auch heute noch in fast allen chirurgischen Fachbereichen für die unterschiedlichen Koagulationen konventionelle Hochfrequenz-Chirurgiegeräte verwendet, welche lediglich Einstellvorrichtungen für die Intensität des HF-Stromes und Schalter zum Ein- und Ausschalten des HF-Stromes haben. Bei Verwendung dieser konventionellen Hochfrequenz-Chirurgiegeräte stellt der Operateur die Intensität des HF-Stromes entsprechend seiner Erfahrung an der Einstellvorrichtung ein und läßt den HF-Strom so lange durch das zu koagulierende Gewebe fließen, bis er den Eindruck hat, die Koagulation sei fertig.

Da die Koagulationsvorgänge in der Regel innerhalb von nur 0,5 bis 5 Sekunden ablaufen und dem Operateur Veränderungen des koagulierenden Gewebes nur während eines Bruchteils dieser relativ kurzen Koagulationszeiten erkennbar werden, ist es praktisch kaum möglich, den Koagulationsvorgang manuell genau in dem Zeitpunkt zu beenden, in welchem die Koagulation optimal ist. Stellt der Operateur die Intensität des HF-Stromes zu gering ein und/oder schaltet er den HF-Strom zu früh ab, so wird die Koagulationstemperatur nicht erreicht und die Koagulation ist insuffizient. Stellt der Operateur die Intensität des HF-Stromes zu hoch ein und/oder schaltet er den HF-Strom zu spät ab, so steigt die Temperatur des Koagulates über die erforderliche Koagulationstemperatur schnell auf über 100°C an, wodurch es sehr rasch zu endogener Dampfbildung innerhalb des Koagulates und hierdurch wiederum zum explosionsartigen Zerplatzen des Koagulates kommen kann, wodurch das Ziel der Koagulation, insbesondere bei der Anwendung der Koagulation zum Verschließen perforierter Blutgefäße, verfehlt wird.

Besonders schwierig sind optimale Koagulationen dann zu erreichen, wenn der Operateur den Koagulationsvorgang nicht beobachten kann, wenn die Koagulation beispielsweise mittels invasiver Koagulationselektroden ausgeführt werden muß, wie beispielsweise bei stereotaktischen Operationen im Bereich der Neurochirurgie oder bei transvenöser Ablatio einer akzessorischen Leiterbahn bei therapierefraktären supraventrikulären Reentry-Tachykardien sowie bei der transabdominalen Tubenligatur.

Es wird seit vielen Jahren versucht, eine Einrichtung zu schaffen, welche den Koagulationsvorgang automatisch beendet, sobald die Koagulation ein definiertes Stadium erreicht hat.

Aus der Deutschen Offenlegungsschrift 312 0102 ist eine Anordnung zur Hochfrequenzkoagulation von Eiweiß für chirurgische Zwecke bekannt, welche eine Abschaltung des HF-Stromes in dem Moment ausführen soll, wenn die Impedanz bzw. der elektrische Widerstand des Koagulates eine bestimmte Veränderung während des Koagulationsvorganges durchläuft.

Es ist seit vielen Jahren bekannt, daß der spezifische elektrische Leitwert elektrolythaltiger biologischer Gewebe weitgehend proportional mit der Temperatur dieses Gewebes zunimmt. Sobald jedoch die Temperatur erreicht wird, bei welcher koagulationsfähige Bestandteile des Gewebes koagulieren, das ist etwa zwischen 50°C und 80°C der Fall, wird die Zunahme des elektrischen Leitwertes als Funktion der Temperatur immer geringer. Oberhalb von 80°C und insbesondere im Bereich des Siedepunktes der intra- und extrazellulären Flüssigkeiten nimmt die elektrische Leitfähigkeit rasch ab. Als Ursache für diese rasche Abnahme der elektrischen Leitfähigkeit oberhalb 80°C vermutet man sowohl die endogene Dampfbildung als auch die Desikkation bzw. Austrocknung des Koagulates.

2

Bei der in der DE-OS 31 20 102 beschriebenen Anordnung zur Hochfrequenzkoagulation von Eiweiß, insbesondere für chirurgische Zwecke, wird laufend die Eiweißimpedanz während der Zuführung von Hochfrequenzleistung bestimmt und der Differentialquotient für die Impedanzkurve nach der Zeit gebildet. Die Werte des Differentialquotienten dienen einmal zur Einregelung der Anfangsleistung, zum anderen zur Feststellung des optimalen Zeitpunkts der Abschaltung der Hochfrequenzleistung. Für beide Situationen sind Voreinstellungen vorgesehen, die die erfindungsgemäße Anordnung flexibel und einfach anpaßbar gestalten. Die Anordnung ist bevorzugt für bipolare Koagultionsinstrumente gedacht; eine Anwendung mit unipolaren instrumenten ist jedoch ebenfalls möglich.

Die Anwendung dieser Anordnung ist insofern problematisch, als die Änderungen der Impedanz des koagulierenden Gewebes durch Schwankungen des Kontaktdruckes zwischen Koagulationselektrode und Gewebe infolge Zittern der Hand des Operateurs und infolge Schrumpfung des koagulierenden Gewebes nicht kontinuierlich ablaufen, sondern mehr oder weniger um einen mittleren Trend schwanken. Hierdurch ergeben sich viele undefinierte Nullstellen des Differentialquotienten. Außerdem ist der Differentialquotient bei langsam ablaufenden Koagulationen infolge der langsamen Änderungen der Eiweißimpedanz derart klein, daß ein definierter Abschaltzeitpunkt nicht gegeben ist.

Es ist Aufgabe der Erfindung, eine Einrichtung für die thermische Koagulation biologischer Gewebe mittels HF-Strom zu schaffen, welche den Koagulationsvorgang automatisch derart kontrolliert, daß verbesserte Operationserfolge sowie eine Entlastung des Chirurgen dadurch erzielt werden können, daß der Koagulationsvorgang in einem genauer definierten Koagulationsstadium automatisch beendet werden kann.

Diese Aufgabe wird erfindungsgemäß durch den Gegenstand des Patent anspruchs 1 gelöst. Vorteilhafte Ausgestaltungen der Erfindung sind Gegenstand der Unteransprüche.

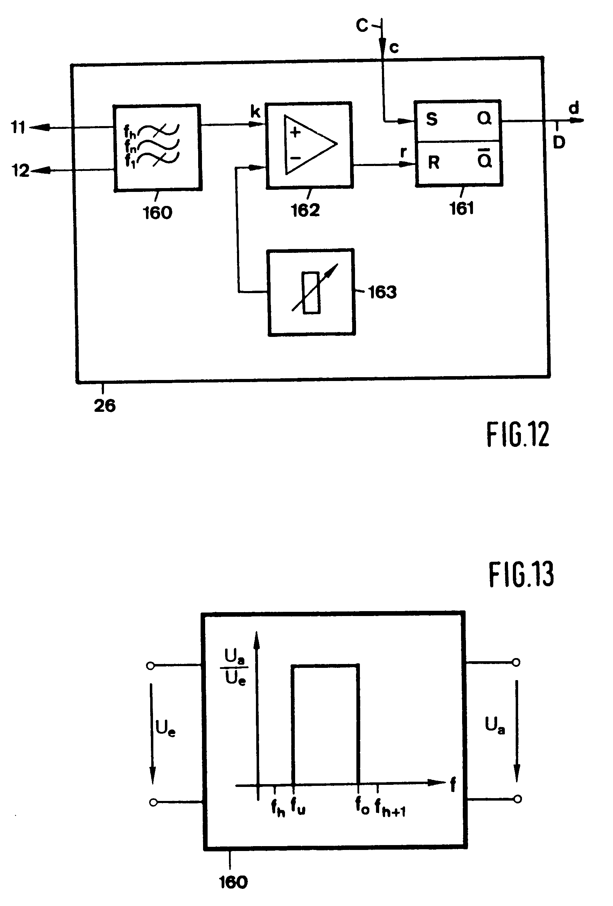

Aus einer älteren, nicht vorveröffentlichten Patentanmeldung der Anmelderin (EP 0 219 568) ist es zwar bereits bekannt, bei einem Hochfrequenz-Chirurgiegerät mit automatischer Regelung der Intensität der elektrischen Lichtbogen zwischen der aktiven Elektrode und dem zu koagulierenden Gewebe einen Lichtbogen-Monitor mit einem Filter entsprechend Fig. 13 vorzusehen, wobei zur Feststellung des Ausmaßes der Lichtbogen ein davon abhängiges elektrisches Signal über das Filter abgeleitet und einem Gleichrichter zugeführt wird, dessen Ausgangssignal einem Regelverstärker zugeführt wird, in welchem dieses mit dem Sollwert eines Sollwertgebers für den Koagulationsvorgang verglichen wird, um ein Signal abzuleiten, welches einem Amplitudenmodulator zugeführt wird, welcher die Amplitude der Ausgangsspannung des Leistungsverstärkers steuert. Dieser älteren Anmeldung kann jedoch weder die Aufgabe der vorliegenden Erfindung noch deren Lösung entnommen werden, in Abhängigkeit von Änderungen der elektrischen Leitfähigkeit des Koagulates in der beschriebenen Weise nichtharmonische Frequenzen zu erfassen, um ein davon abhängiges Signal zu erzeugen, mit dem der Koagulationsvorgang in einem genau definierten Koagulationsstadium beendet werden kann. Deshalb sind auch die für diese Zielsetzung geeigneten Mittel in der älteren Anmeldung nicht genannt.

Dies gilt auch für die im Patentanspruch 2 beanspruchte vorteilhafte Weiterbildung der Erfindung, die Gegenstand des Patentanspruchs 1 des Stammpatents für eine automatische Steuerung des Koagulationsvorganges sind.

Die Erfindung wird im folgenden anhand von Ausführungsbeispielen und Zeichnungen detaillierter beschrieben. In den Zeichnungen sind identische Elemente Identisch gekennzeichnet. Unterstrichene Elemente sind in anderen Zeichnungen detaillierter dargestellt. Es zeigen:

Figur 1      einen typischen Verlauf der Amplitudenänderungen des HF-Stromes während eines Koagulationsvorganges.

Figur 2      einen Strommonitor, mittels welchem der Verlauf der Amplitudenänderungen des HF-Stromes während eines Koagulationsvorganges überwacht und ein definierter Abschaltzeitpunkt ermittelt wird.

Figur 3      ein Diagramm, welches den Verlauf der elektrischen Spannungen $U_a$ und $kU_b$ während eines Koagulationsvorganges zeigt.

Figur 4      ein Blockschaltbild eines Ausführungsbeispieles der erfindungsgemäßen Einrichtung zur thermischen Koagulation biologischer Gewebe mittels HF-Strom.

Figur 5      ein Blockschaltbild einer weiteren Ausgestaltung der erfindungsgemäßen Einrichtung entsprechend der Figur 4.

Figur 6      ein Blockschaltbild eines Ausführungsbeispieles der Steuerung der Leerlaufspannung $U_o$ des Hochfrequenzgenerators.

Figur 7      ein Ausführungsbeispiel des HF-Strom-Indikators.

Figur 8      ein Ausführungsbeispiel eines akustischen Signalgebers, welcher den Operateur über den Koagulationsvorgang informiert.

Figur 9      ein Ausführungsbeispiel eines weiteren akustischen Signalgebers, welcher den Operateur über Fehlfunktionen der Einrichtung informiert.

Figur 10 ein Ausführungsbeispiel einer automatischen Schaltvorrichtung.

Figur 11 ein Ausführungsbeispiel einer Schaltlogik.

Figur 12 ein Ausführungsbeispiel eines Lichtbogenmonitors.

Figur 13 ein Diagramm einer Filtercharakteristik des Filters 160.

Figur 14 ein Blockschaltbild eines Ausführungsbeispieles der erfindungsgemäßen Einrichtung zur thermischen Koagulation biologischer Gewebe mittels HF-Strom.

Stromes während eines Koagulationsvorganges dargestellt, aus welchem ein definiertes Signal zum Abschalten des HF-Stromes hergeleitet werden soll.

Im Zeitpunkt $t_1$, in weichem der HF-Strom durch das zu koagulierende biologische Gewebe zu fließen beginnt, erreicht die Amplitude A des HF-Stromes in Abhängigkeit von den oben genannten Parametern die Amplitude $A_1$. Infolge Erwärmung des biologischen Gewebes, dessen elektrische Leitfähigkeit durch die in ihm enthaltenen Elektrolyte verursacht wird, steigt die elektrische Leitfähigkeit und damit die Amplitude A des HF-Stromes an. Sobald die Temperatur des biologischen Gewebes auf etwa 70°C angestiegen ist, koagulieren die koagulationsfähigen Bestandteile des biologischen Gewebes, wodurch dessen elektrische Leitfähigkeit abnimmt, so daß die Amplitude A des HF-Stromes von der maximal erreichten Amplitude $A_2$ im Zeitpunkt $t_2$ mehr oder weniger schnell kleiner wird. Beschleunigt wird das Abfallen der Amplitude des HF-Stromes in Abhängigkeit von der Temperatur des Koagulates durch das Verdampfen der Gewebeflüssigkeiten, wodurch das Koagulat austrocknet. Sobald die Austrocknung des Koagulates, die auch Desikkation genannt wird, ein bestimmtes Stadium erreicht hat, bei welchem die Amplitude A des HF-Stromes auf einen vernachlässigbaren Pegel $A_3$ abgefallen ist, kommt der Koagulationsvorgang zum Stillstand.

Entscheidend für das Auffinden einer geeigneten Lösung für die automatische Abschaltung des HF-Stromes in einem definierten Zeitpunkt des Koagulationsvorganges aus den Änderungen der Amplitude des HF-Stromes bzw. aus den Änderungen der Impedanz des Koagulates ist die Beachtung der unstetigen Veränderungen der Amplitude A(t) des HF-Stromes während des Koagulationsvorganges. Wie bereits oben bei der Darstellung des Standes der Technik angeführt, schwankt die Amplitude A(t) des HF-Stromes während eines jeden Koagulationsvorganges mehr oder weniger stark um den mittleren Trend a(t) der Amplitude des HF-Stromes. Diese mehr oder weniger starken Schwankungen der Amplitude des HF-Stromes während eines jeden Koagulationsvorganges resultiert insbesondere aus dem ungleichmäßigen Kontaktdruck der Koagulationselektrode gegen das zu koagulierende Gewebe infolge unkontrollierter Bewegungen der Hand des Operateurs und/oder aus der Schrumpfung des koagulierenden und austrocknenden Gewebes.

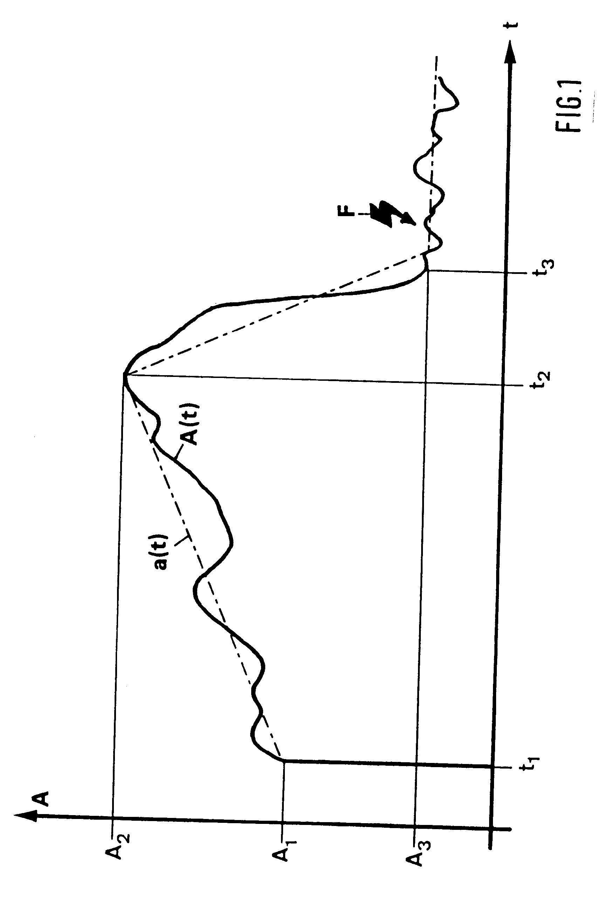

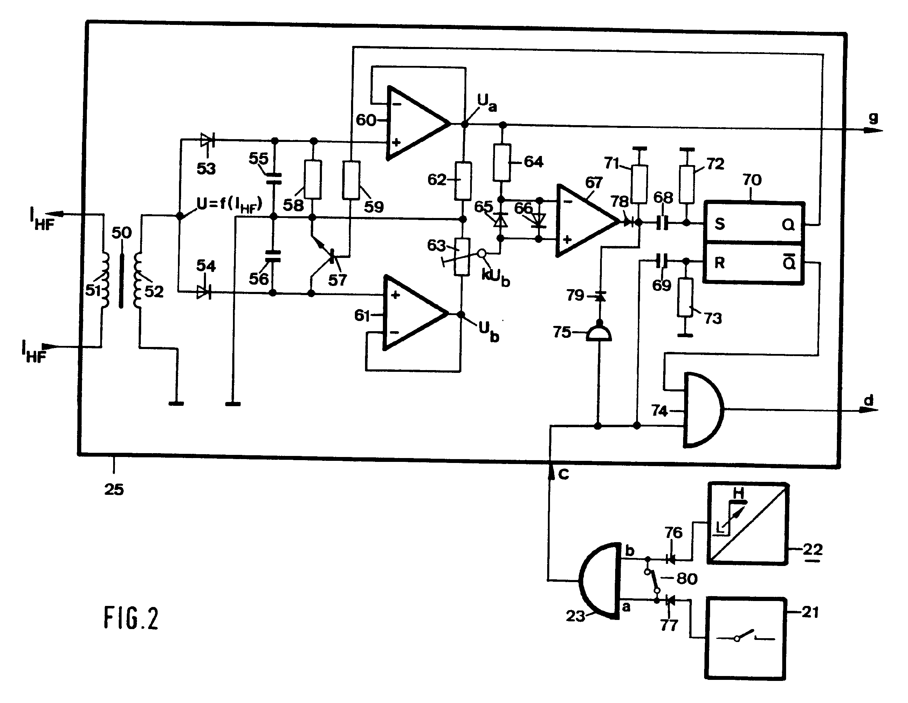

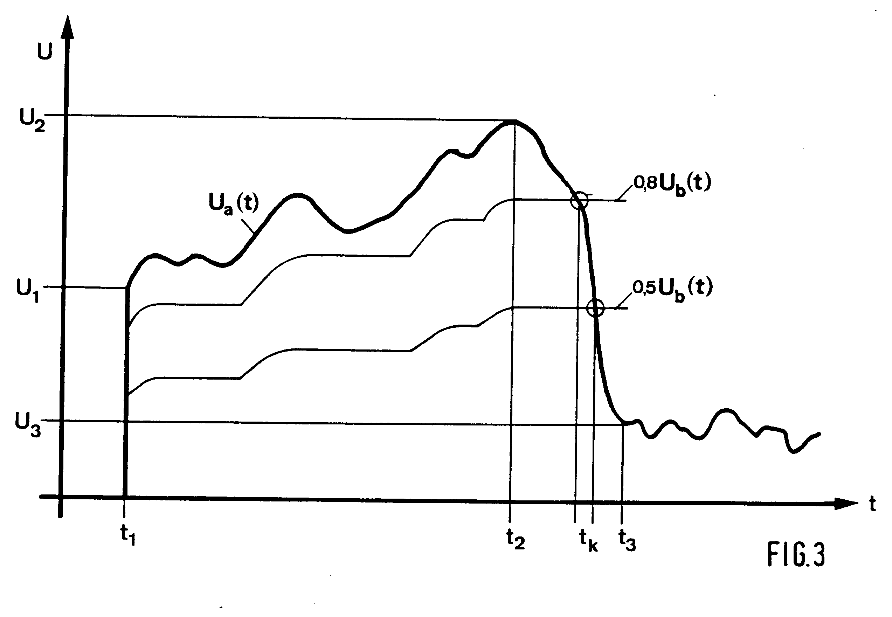

Bekannte Einrichtungen für die thermische Koagulation biologischer Gewebe, welche ein definiertes Abschaltkriterium aus den Änderungen der Amplitude des HF-Stromes bzw. aus den Änderungen der Impedanz des Koagulates abzuleiten versuchen, vernachlässigen die oben beschriebenen Unstetigkeiten der Änderungen der Amplitude A(t) und gehen von der praktisch nur selten gegebenen Änderung a(t) aus, welche den theoretisch gemittelten Trend der in der Regel unstetigen Änderung A(t) darstellt. Wie aus dem in Figur 1 dargestellten Beispiel eines realistischen Verlaufes der Amplitudenänderungen A(t) über der Zeit t erkennbar, liefert der Differentialquotient dA(t)/dt kein eindeutiges Abschaltkriterium für den HF-Strom. Eine für die automatische Abschaltung des HF-Stromes und damit Beendigung des Koagulationsvorganges geeignete Vorrichtung wird anhand der Figuren 2 und 3 beschrieben.

Ein weiteres Kriterium, welches am Ende eines Koagulationsvorganges zur automatischen Abschaltung des HF-Stromes genutzt werden kann, ist die Zündung elektrischer Lichtbogen bzw. Funken zwischen der Koagulationselektrode und dem Koagulat. Das Entstehen elektrischer Lichtbogen bzw. Funken, im folgenden nur elektrische Lichtbogen genannt, am Ende von Koagulationsvorgängen ist bereits seit vielen Jahren bekannt. Es fehlte bisher jedoch an geeigneten Methoden und Mitteln, das Zünden und Vorhandensein elektrischer Lichtbogen zwischen der Koagulationselektrode und dem Koagulat in geeigneter Weise elektronisch zu überwachen und hieraus geeignete Abschaltsignale herzuleiten. Anhand der Figuren 12 und 13 wird ein für die automatische Abschaltung des HF-Stromes geeigneter Lichtbogenmonitor beschrieben.

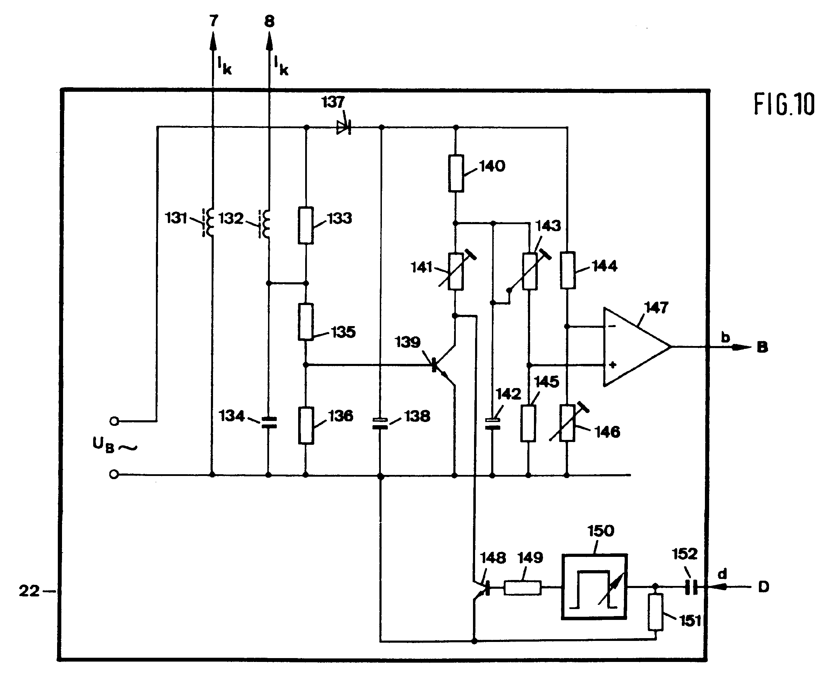

Elektrische Lichtbogen entstehen in der Regel ab dem Zeitpunkt $t_3$. Sie können jedoch auch bereits vor dem Zeitpunkt $t_3$ entstehen, und zwar wenn die Amplitude $A_1$ des HF-Stromes im Zeitpunkt $t_1$ bereits so groß ist, daß im Grenzbereich zwischen der Koagulationselektrode und dem Gewebe eine derart hohe elektrische Feldstärke vorhanden ist, daß elektrische Lichtbogen zünden können. Letzteres ist zB. dann gegeben, wenn der HF-Generator eingeschaltet wird, bevor die Koagulationselektrode das Gewebe elektrisch leitfähig berührt und die Koagulationselektrode im Zeitpunkt $t_1$ bei bereits eingeschaltetem HF-Generator das Gewebe berührt. Es ist deswegen eine Vorrichtung erforderlich, welche das Einschalten des HF-Generators verhindert, wenn die Koagulationselektrode keinen elektrisch leitfähigen Kontakt zum Gewebe hat. Eine hierfür geeignete Vorrichtung wird anhand der Figur 10 weiter unten beschrieben.

Die Zündung elektrischer Lichtbogen vor $t_3$ oder gar vor $t_2$ ist auch dann möglich, wenn die Koagulationselektrode das Gewebe zwar elektrisch leitfähig berührt bevor der HF-Generator eingeschaltet wird, die An-

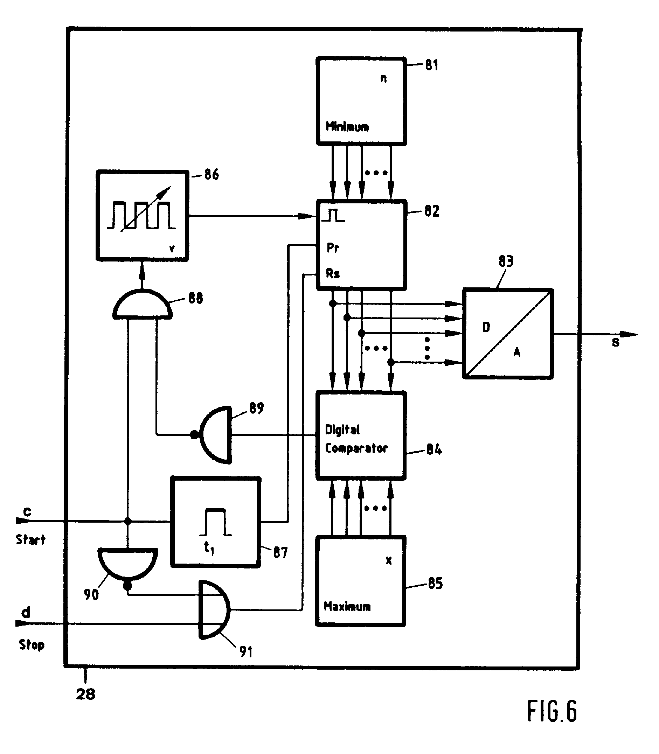

fangsamplitude $A_1$ des HF-Stromes im Einschaltzeitpunkt $t_1$ jedoch zu hoch ist. Es ist deswegen eine Vorrichtung erforderlich, welche die Amplitude des HF-Stromes so steuert, daß die Anfangsamplitude $A_1$ im Einschaltzeitpunkt $t_1$ nicht zu groß ist und im Zeitintervall $t_1$ bis $t_2$ oder $t_3$ zweckmäßig gesteuert wird. Eine hierfür geeignete Vorrichtung wird anhand der Figur 6 weiter unten beschrieben.

In Figur 2 sind die erfindungsrelevanten Details eines Ausführungsbeispieles eines Strommonitors 25 dargestellt. Mittels eines Strom/Spannungswandlers 50 bis 52 wird eine dem HF-Strom $I_{HF}$ proportionale Spannung $U = f (I_{HF})$ gebildet, welche zwei unterschiedlich wirkenden Detektoren zugeführt wird.

Ein erster Detektor, bestehend aus einer Diode 53, einem Kondensator 55, einem Widerstand 58, einem Spannungsfolger 60 und einem Widerstand 62 ist so zu dimensionieren, daß seine Ausgangsspannung $U_a$ möglichst schnell den Amplitudenschwankungen der Spannung $U = f (I_{HF})$ folgt. Die Zeitkonstante der Parallelschaltung des Kondensators 55 mit dem Widerstand 58 ist vorzugsweise so zu bemessen, daß einerseits die Grundfrequenz des HF-Stromes, beispielsweise 500 kHz, am Eingang des Spannungsfolgers 60 unterdrückt wird, andererseits Amplitudenschwankungen des HF-Stromes möglichst ungedämpft im Ausgangssignal $U_a$ des Spannungsfolgers 60 erscheinen. Bei der Bemessung dieser Zeitkonstante muß auch die Modulation des HF-Stromes $I_{HF}$ infolge sogenanntem Netzbrummen beachtet werden. Netzbrumm meint hier die Amplitudenschwankungen des HF-Stromes $I_{HF}$ infolge fehlender oder unvollständiger Glättung der Betriebsspannung des Hochfrequenzgenerators, so daß die Amplitude des HF-Stromes $I_{HF}$ mehr oder weniger stark mit der doppelten Netzfrequenz moduliert ist. Ist in dem HF-Strom $I_{HF}$ ein zu starker Netzbrumm enthalten, so muß die Zeitkonstante der Elemente 55 und 58 so bemessen werden, daß dieser Netzbrumm am Eingang des Spannungsfolgers 60 ausreichend unterdrückt wird, was allerdings den Frequenzgang des ersten Detektors verschlechtert. Es ist deswegen empfehlenswert, die Versorgungsspannung des Hochfrequenzgenerators möglichst gut zu glätten.

Ein zweiter Detektor, bestehend aus einer Diode 54, einem Kondensator 56, einem Spannungsfolger 61 und einem Spannungsteiler 63 ist so zu dimensionieren, daß er als Spitzenwertdetektor wirkt, so daß die Ausgangsspannung $U_b$ proportional zu den Spitzenwerten der Spannung $U = f (I_{HF})$ ansteigt.

Die Spannung $U_a$ und die um einen einstellbaren Faktor k, welcher kleiner als 1 sein muß, heruntergeteilte Spannung $kU_b$ werden gleichzeitig einem Spannungskomparator 67 zugeleitet. Die beiden Dioden 65 und 66 sichern in bekannter Weise den Eingang des Spannungskomparators gegen zu hohe Spannungsdifferenzen.

Anhand des Diagrammes der Figur 3 soll die Wirkungsweise der beiden oben beschriebenen Detektoren und des Spannungskomparators im Strommonitor 25 erklärt werden. Die Kurve $U_a (t)$ stellt die Ausgangsspannung $U_a$ über der Zeit t während eines Koagulationsvorganges dar. Diese Kurve folgt weitgehend proportional den Amplitudenschwankungen $A (t)$ des HF-Stromes $I_{HF}$. In dasselbe Diagramm sind beispielsweise zwei verschiedene Kurven eingetragen, welche die um einen einstellbaren Faktor k heruntergeteilte Spannung $kU_b (t)$ wiedergeben. Für die eine Kurve wurde k = 0,8, für die andere Kurve k = 0,5 eingestellt. Je nach der Einstellung des Faktors k schneidet die Kurve $kU_b (t)$ die Kurve $U_a (t)$ innerhalb des Zeitintervalles $t_2$ bis $t_3$ früher oder später. Der Spannungskomparator 67 liefert im Zeitpunkt $t_k$ an seinem Ausgang einen positiven Spannungssprung, sobald die Spannung $U_a (t)$ kleiner wird als $kU_b (t)$, welcher eine bistabile Kippstufe 70, beispielsweise ein RS-Flip-Flop, über den Kondensator 68 dynamisch so setzt, daß dessen Ausgangssignal $\overline{Q}$ = Null wird. Gleichzeitig wird der Kondensator 56 des Spitzenwertdetektors durch den Transistor 57 entladen, wofür das Ausgangssignal Q = 1 des RS-Flip-Flop benutzt wird.

Das Ausgangssignal d des Strommonitors 25 schaltet den HF-Strom $I_{HF}$ ein oder aus, wobei dieses Ausgangssignal d von der UND-Bedingung 74 der Signale $\overline{Q}$ des RS-Flip-Flop 70 und des Signales c aus den Schaltvorrichtungen 21 und/oder 22 abhängig ist.

Die bistabile Kippstufe 70 bleibt nach jeder automatischen Abschaltung eines Koagulationsvorganges im Zustand Q = 1 und $\overline{Q}$ = 0, bis das Signal c infolge manueller 21 oder automatischer 22 Einschaltung von logisch 0 auf 1 springt und sie über den Kondensator 69 zurücksetzt.

Wird während des Koagulationsvorganges das Signal c infolge vorzeitigen Abschaltens der manuellen 21 und/oder automatischen 22 Schaltvorrichtung von logisch 1 auf 0 geschaltet, so wird die bistabile Kippstufe 70 durch das im NICHT-Glied 75 invertierte Signal $\overline{c}$ so gesetzt, daß Q = 1 wird und der Kondensator 56 über den Transistor 57 entladen wird. Die übrigen Elemente der Figur 2 sind bezüglich ihrer Funktion dem Fachmann bekannt oder werden weiter unten beschrieben.

Statt der automatischen Herleitung eines definierten Abschaltzeitpunktes $t_k$ aus den Amplitudenänderungen des HF-Stromes $I_{HF}$ kann dieser Abschaltzeitpunkt auch aus den Amplitudenänderungen der HF-Spannung $U_{HF}$ am Ausgang 11, 12 der Einrichtung hergeleitet werden. Hierzu kann der gleiche Strommonitor 25 verwendet werden, wobei jedoch der Strom/Spannungswandler 50 bis 52 entfällt und statt dessen ein HF-Gleichrichter bzw. Demodulator, welcher die HF-Spannung $U_{HF}$ gleichrichtet bzw. demoduliert und ein Inverter, welcher die so gewonnene Gleichspannung invertiert, erforderlich ist. Mit Inverter ist hier eine Einrichtung gemeint, welche einen ansteigenden Spannungspegel in einen abfallenden Spannungspegel und einen abfallenden Spannungspegel in einen ansteigenden Spannungspegel invertiert. Die auf diese Weise demodulierte und invertierte HF-Spannung $U_{HF}$ kann direkt den beiden Detektoren des Strommonitors 25 zugeführt werden.

Außerdem kann ein definierter Abschaltzeitpunkt $t_k$ aus der Änderung des elektrischen Leitwertes des Gewebes zwischen der monopolaren Koagulationselektrode 15 und der Neutralelektrode 18 bzw. zwischen den beiden Polen einer bipolaren Koagulationselektrode 13 hergeleitet werden. Hierzu wird der Leitwert aus den Amplituden der Spannung $U_{HF}$ und des Stromes $I_{HF}$ elektronisch berechnet, beispielsweise mittels bekannter analoger Dividierschaltungen. Die dem Leitwert proportionale Ausgangsspannung des elektronischen Analogdividierers kann wie die Spannung $U = f ( I_{HF} )$ den beiden Detektoren des Strommonitors 25 zugeführt werden.

Die Herleitung des Abschaltzeitpunktes $t_k$ aus den Änderungen des elektrischen Leitwertes des Gewebes während des Koagulationsvorganges ist zwar aufwendiger als dessen Herleitung aus den Amplitudenänderungen des HF-Stromes $I_{HF}$ oder der HF-Spannung $U_{HF}$, bietet jedoch den Voteil, daß Amplitudenschwankungen, welche vom HF-Generator verursacht werden, beispielsweise infolge Netzbrumm oder Netzspannungsschwankungen, automatisch eleminiert werden.

Statt des in Figur 3 dargestellten Strom/Spannungswandlers 50 bis 52 sind für diesen Zweck auch andere Strom/Spannungswandler verwendbar, wie beispielsweise photoelektrische oder thermoelektrische Strom/Spannungswandler. Vorzugsweise sollten solche Strom/Spannungswandler verwendet werden, welche schnell genug reagieren, eine Potentialtrennung mit möglichst hoher Spannungsfestigkeit zwischen Eingang 51 und Ausgang 52 haben und eine möglichst geringe kapazitive Kopplung zwischen Eingang und Ausgang haben.

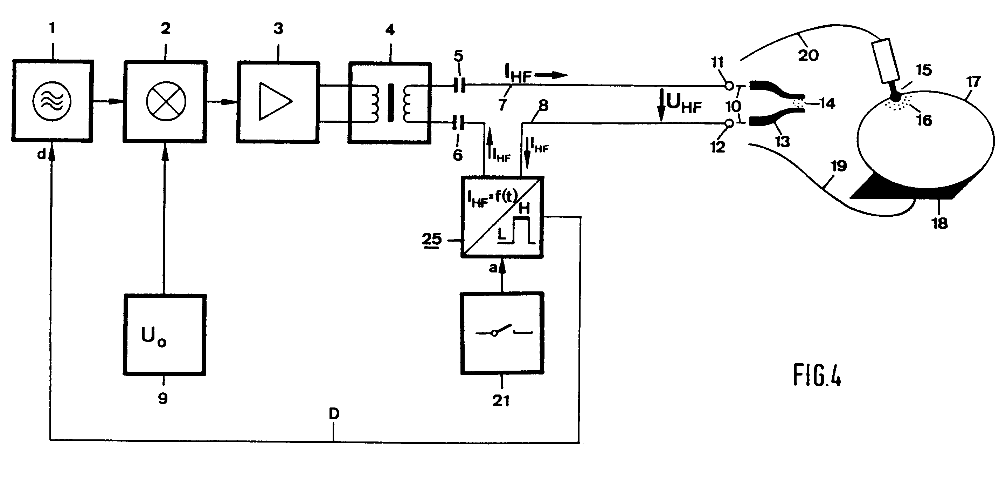

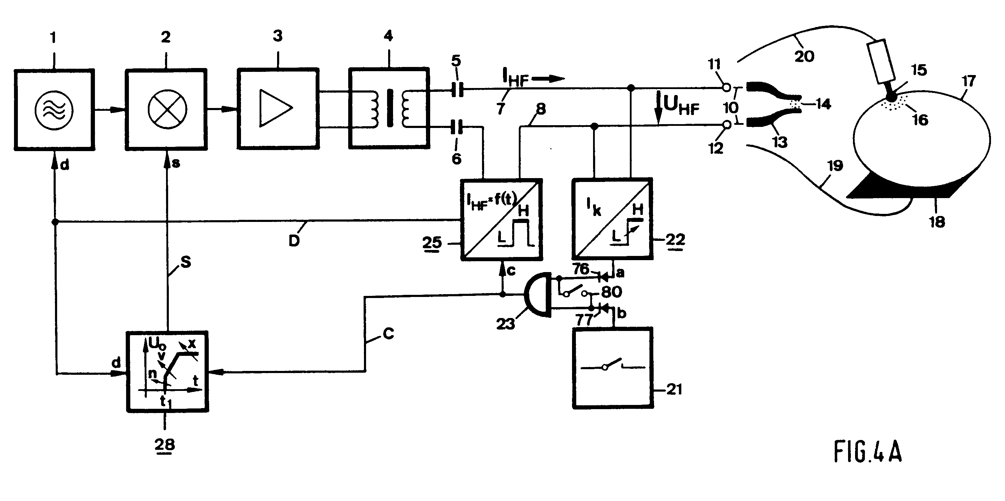

Anhand eines Blockschaltbildes in Figur 4 soll ein Ausführungsbeispiel einer erfindungsgemäßen Einrichtung für die thermische Koagulation biologischer Gewebe mittels HF-Strom beschrieben werden.

Der Hochfrequenzgenerator, welcher den HF-Strom $I_{HF}$ für den Koagulationsvorgang generiert, besteht aus einem HF-Oszillator 1, einem Amplitudenmodulator 2, einem Leistungsverstärker 3 und einem Ausgangstransformator 4.

Die Amplitude A des HF-Stromes $I_{HF}$ ergibt sich aus der Leerlaufspannung $U_o$ des Hochfrequenzgenerators und der Summe aller Widerstände im Stromkreis. Die Leerlaufspannung $U_o$ kann in bekannter Weise an einer Einstellvorrichtung 9 eingestellt werden und wird durch den Amplitudenmodulator 2 realisiert. Der Amplitudenmodulator 2 ist beispielsweise ein Vorverstärker, dessen Verstärkungsfaktor an der Einstellvorrichtung 9 einstellbar ist. Da der elektrische Widerstand des zu koagulierenden Gewebes und insbesondere der Betrag der Widerstandsänderungen des zu koagulierenden Gewebes während der Koagulationsvorgänge im Vergleich zur Summe aller den HF-Strom $I_{HF}$ bestimmenden Teilwiderstände möglichst groß sein sollte, damit diese Widerstandsänderungen vom Strommonitor 25 problemlos erkannt werden, ist darauf zu achten, daß alle anderen Teilwiderstände, insbesondere der Innenwiderstand des Hochfrequenzgenerators, möglichst klein sind, beispielsweise 50 Ohm für bipolare und 200 Ohm für monopolare Koagulationen. Nach Einschalten der manuellen Einschaltvorrichtung 21 wird durch das Signal c, welches beispielsweise aus logischen Spannungspegeln (L = Low, H = High) besteht, der Strommonitor 25 aktiviert, der gleichzeitig durch das Signal d über die Leitung D den HF-Oszillator einschaltet.

Die Funktion des in der Figur 2 detailliert dargestellten Strommonitors 25 wurde bereits oben beschrieben, lediglich mit dem Unterschied, daß in dem Ausführungsbeispiel entsprechend der Figur 4 nur eine manuelle Schaltvorrichtung 21 verwendet ist. Selbstverständlich kann das in Figur 4 dargestellte Ausführungsbeispiel alternativ oder zusätzlich mit einer automatischen Schaltvorrichtung 22 ausgestattet werden. Automatische Schaltvorrichtungen sind aus der DAS 1099 658, DP 2540 968 und DE 2946 728 bekannt.

Sobald das anhand der Figuren 2 und 3 beschriebene Abschaltkriterium erreicht ist, wird der HF-Oszillator 1 automatisch durch das Signal d über die Leitung D abgeschaltet, und zwar so lange, bis die Schaltvorrichtung 21 erneut betätigt wird.

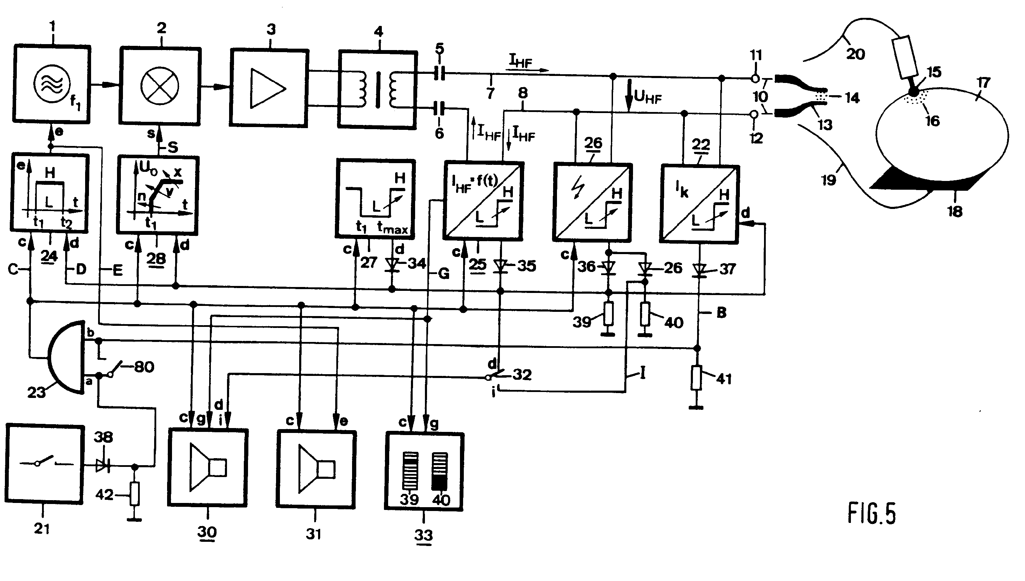

Anhand der Figuren 5 bis 12 werden zweckmäßige Weiterentwicklungen des anhand der Figur 4 beschriebenen Ausführungsbeispieles einer Einrichtung zur thermischen Koagulation biologischen Gewebes mittels HF-Strom erklärt.

Figur 5 zeigt ein Blockschaltbild über das Zusammenwirken aller weiter unten detaillierter beschriebenen Zusatzvorrichtungen, welche das in Figur 4 dargestellte Ausführungsbeispiel entsprechend der Aufgabe dieser Erfindung weiter verbessern.

Eine zweckmäßige Verbesserung des anhand der Figur 4 beschriebenen Ausführungsbeispieles ist die Steuerungseinrichtung 28, welche die Leerlaufspannung $U_o$ des Hochfrequenzgenerators über den Amplitudenmodulator 2 von einem einstellbaren Minimum-Pegel n mit einstellbarer Geschwindigkeit v auf einen einstellbaren Maximum-Pegel x steuert. Diese Steuerungseinrichtung wird vom Einschaltsignal c über die Leitung C so getriggert, daß der einstellbare Minimum-Pegel n im Zeitpunkt $t_1$ der Einschaltung des Oszillators 1 beginnt. Wird der Minimum-Pegel n auf Null eingestellt, so steigt die Spannung $U_o$ ab dem Zeitpunkt $t_1$ von

Null aus an. Ist die monopolare Koagulationselektrode 15 oder die beiden Pole der bipolaren Koagulationselektrode 13 mit dem Gewebe des Patienten 17 im elektrisch leitfähigen Kontakt, so steigt auch der Strom $I_{HF}$ von Null aus an, Hierdurch ist es möglich, beliebig große oder kleine Koagulationselektroden zu verwenden ohne die bei konventionellen HF-Chirurgiegeräten erforderliche, der jeweiligen Größe der Koagulationselektrode entsprechende HF-Leistung voreinzustellen. Sobald das anhand der Figuren 2 und 3 beschriebene Abschaltkriterium erreicht ist, wird der Koagulationsvorgang automatisch beendet. Für große Koagulationen, bei denen nur größere Koagulationselektroden verwendet werden, ist es vorteilhaft, einen höheren Minimum-Pegel n einzustellen. Die Steuerungseinrichtung 28 wird weiter unten anhand eines Ausführungsbeispieles detaillierter beschrieben.

Um zu verhindern, daß die Steuerungseinrichtung 28 die Leerlaufspannung $U_o$ des HF-Generators bereits hochsteuert, bevor die Koagulationselektrode 15 oder 13 das zu koagulierende Gewebe elektrisch leitfähig berührt, ist eine automatische Schaltvorrichtung 22 vorhanden, welche über eine UND-Verknüpfung 23 die Einschaltung des Oszillators 1 und die Triggerung der Steuerungseinrichtung 28 solange verhindert wenn der Schalter 80 geöffnet ist, bis entweder die monopolare Koagulationselektrode 15 gleichzeitig mit der Neutralelektrode 18 oder beide Pole der bipolaren Koagulationselektrode 13 das Gewebe des Patienten 17 gleichzeitig elektrisch leitfähig berühren. Ein Ausführungsbeispiel einer für diesen Zweck geeigneten automatischen Schaltvorrichtung 22 wird weiter unten anhand der Figur 10 detaillierter beschrieben. Um die Redundanz der automatischen Abschaltfunktion zu erhöhen, was mit Rücksicht auf die automatische Steuerung 28 der Leer-laufspannung $U_o$ des HF-Generators insofern wichtig ist, als ein Ausbleiben der Abschaltfunktion des Strommonitors 25 dazu führen würde, daß die optimale Koagulation, insbesondere bei Verwendung kleinerer Koagulationselektroden, schneller als bei konventionellen HF-Chirurgiegeräten, welche eine weitgehend kon-stante Spannung $U_o$ liefern, überschritten würde, ist eine weitere automatische Abschaltvorrichtung, der Lichtbogenmonitor 26, vorhanden, der im Falle eines Ausbleibens der Abschaltfunktion des Strommonitors 25 die Abschaltung in dem Moment durchführt, in dem ein elektrischer Lichtbogen zwischen der Koagulationselektrode 15 oder 13 und dem Gewebe des Patienten 17 entsteht. Ein für diesen Zweck geeigneter Lichtbogenmonitor 26 wird anhand der Figuren 12 und 13 detaillierter beschrieben.

Für den Fall, daß eines der oder beide oben beschriebene automatische Abschaltkriterien ausbleiben, ist eine zwangsweise Abschaltung mittels eines Einschaltdauer-Begrenzers 27 vorhanden, welcher den HF-Generator nach Ablauf einer voreinstellbaren Dauer $t_{max}$ abschaltet. Dieser Einschaltdauer-Begrenzer 27 ist beispielsweise ein durch einen Logigpegel c triggerbarer elektrischer Timer, der nach Ablauf einer einstellbaren Dauer $t_{max}$ ein Signal d auf die Leitung D liefert und hierdurch den HF-Generator abschaltet sowie die Einrichtungen 28, 22, 24, und 30 zurücksetzt.

Dieser Einschaltdauer-Begrenzer 27 muß so ausgestattet sein, daß jeder Triggerimpuls c das Zeitintervall $t_1$ bis $t_{max}$ neu startet. Eine für diesen Zweck geeignete Einschaltdauer-Begrenzung ist beispielsweise ein nachtriggerbarer Zeitschalter bzw. nachtriggerbares Monoflop (Literatur: U. Tietze, Ch. Schenk: Halbleiter-Schaltungstechnik, 5. Aufl., Seite 448 ff, Verlag Springer, Berlin, Heidelberg, New York, 1980).

Die Einrichtung entsprechend Figur 5 kann wahlweise manuell durch die manuelle Schaltvorrichtung 21 und/oder automatisch durch die automatische Schaltvorrichtung 22 eingeschaltet werden, je nachdem, ob der Schalter 80 geschlossen oder offen ist.

Ist der Schalter 80 geschlossen, so kann die Einrichtung entweder manuell oder automatisch eingeschaltet werden. Ist der Schalter 80 geöffnet, so kann die Einrichtung manuell nur dann eingeschaltet werden, wenn die Bedingungen der automatischen Schaltvorrichtung 22 erfüllt sind, d.h., wenn die monopolare Koagulationselektrode 15 und die Neutralelektrode 18 oder beide Pole der bipolaren Koagulationselektrode 13 gleichzeitig das Gewebe des Patienten 17 elektrisch leitfähig berühren. Durch diese UND-Verknüpfung der beiden Schaltvorrichtungen 21 und 22 wird die oben beschriebene Bedingung für die automatische Steuerungseinrichtung 28 erfüllt, daß die Spannung $U_o$ erst dann einschaltbar ist, wenn die Koagulationselektroden das Gewebe elektrisch leitfähig berühren. Umgekehrt kann die automatische Schaltvorrichtung 22 die Einrichtung nur dann automatisch einschalten, wenn die manuelle Schaltvorrichtung 21 betätigt ist. Diese UND-Verknüpfung der beiden Schaltvorrichtungen 21 und 22 verhindert eine unbeabsichtigte Einschaltung der Einrichtung, wenn beispielsweise die bipolare Koagulationselektrode unbeabsichtigt das Gewebe des Patienten berührt.

Anhand der Figur 6 wird im folgenden ein Ausführungsbeispiel einer Steuerungseinrichtung 28 für die au-tomatische Steuerung der Leerlaufspannung $U_o$ des HF-Generators beschrieben.

Die automatische Abschaltung des HF-Stromes $I_{HF}$ und damit die automatische Beendigung des Koagulationsvorganges bei Erreichen eines definierten Koagulationsstadiums, bei welchem entweder die Änderung der Amplitude des HF-Stromes $I_{HF}$ und/oder die Zündung elektrischer Lichtbogen zwischen der Koagulationselektrode und dem Gewebe als Abschaltkriterium genutzt werden, ermöglicht und fordert eine automatische Steuerung der Koagulationsleistung $P_{HF} = U_{HF}^2 / R$, wobei R den reellen elektrischen Lastwiderstand an den Ausgangsklemmen 11 und 12 meint, beispielsweise durch Steuerung der Leerlaufspannung $U_o$ des HF-Generators, so daß die Koagulationsleistung $P_{HF}$ sich in Abhängigkeit von der Leerlaufspannung $U_o$ entsprechend folgender Gleichung ergibt:

$$P_{HF} = \left(\frac{U_o}{R_i + R_a}\right)^2 \cdot R_a$$

hierin ist $R_i$ der Innenwiderstand des HF-Generators und $R_a$ der reelle Lastwiderstand an den Ausgangsklemmen 11 und 12. Steigt die Koagulationsleistung $P_{HF}$ von einem Minimum-Pegel mehr oder weniger schnell, beispielsweise von Null aus an, so wird dank der automatischen Abschaltung des HF-Stromes $I_{HF}$ stets eine optimale Koagulation unabhängig davon erreicht, ob eine sehr kleine oder eine große Menge Gewebe 14 zwischen den Polen der bipolaren Koagulationselektrode 13 gefaßt ist und unabhängig davon, ob feine oder grobe Koagulationselektroden verwendet werden.

Die Leerlaufspannung $U_o$ soll automatisch von einem voreinstellbaren Minimum-Pegel $U_{min}$ mit einstellbarer Geschwindigkeit v auf einen voreinstellbaren Maximum-Pegel $U_{max}$ gesteuert werden. Das Ausführungsbeispiel ist in Digitaltechnik realisiert. Der Minimum-Pegel $U_{min}$ wird an einem digitalen Vorwahlschalter 81 eingestellt und als digitale Zahl n einem programmierbaren Zähler 82 zugeführt. Sobald das Signal e = Start High-Pegel annimmt, was dem Zeitpunkt $t_1$ entspricht, wird der Zähler 82 über den Preset-Eingang Pr durch einen kurzen Impuls, der von einem Monoflop 87 gebildet wird, gestartet und übernimmt die Digitalzahl n aus dem Vorwahlschalter 81. Das Signal e startet über das UND-Glied 88 den Impulsgenerator 86, dessen Impulsfrequenz einstellbar ist. Der Zähler 82 zählt von der am Vorwahlschalter 81 voreingestellten Zahl n beginnend die Impulse des Impulsgenerators 86 aufwärts oder abwärts, je nachdem, ob die dem Maximum-Pegel entsprechende digitale Zahl x des Vorwahlschalters 85 kleiner oder größer als die dem Minimum-Pegel entsprechende digitale Zahl n definiert wird.

Der Ausgang des Zählers 82 wird auf einen digitalen Komparator 84 geführt, welcher die digitale Zahl des Zählers 82 mit der digitalen Zahl x eines Vorwahlschalters 85, an welchem der Maximum-Pegel der Leerlaufspannung $U_{max}$ voreingestellt wird, vergleicht. Sobald die digitale Zahl am Ausgang des Zählers 82 gleich der digitalen Zahl x des Vorwahlschalters 85 ist, liefert der digitale Komparator 84 ein Ausgangssignal an den Impulsgenerator 86, welches den Impulsgenerator abschaltet. Wird ein Digital-Komparator verwendet, welcher ab Gleichstand beider digitaler Zahlen an seinem Ausgang einen High-Pegel liefert, so muß dieses Signal mittels eines NICHT-Gliedes 89 invertiert werden. An den Ausgang des Zählers 82 ist ein Digital/Analog-Wandler angeschlossen, der aus der digitalen Zahl einen analogen Spannungspegel s bildet, welcher über die Leitung S dem Amplitudenmodulator 2 zugeführt wird. Sobald das Signal e infolge Abschaltung der manuellen 21 oder automatischen 22 Schaltvorrichtung von High- auf Low-Pegel oder das Signal d von Low- auf High-Pegel wechselt, wird der Zähler 82 über seinen Reset-Eingang Rs über das ODER-Glied 91 auf Null zurückgesetzt. Das Signal e wird hierfür im NICHT-Glied 90 invertiert.

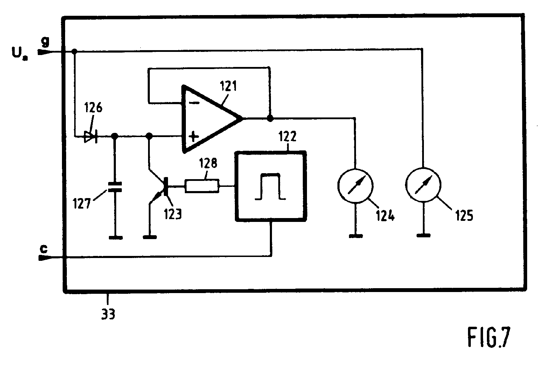

Anhand der Figur 7 wird im folgenden ein Ausführungsbeispiel des HF-Strom-Indikators 33 detaillierter beschrieben.

Der HF-Strom-Indikator 33 zeigt dem Operateur auf einem analogen oder digitalen Anzeigeinstrument 125 den jeweiligen Momentanwert des Stromes $I_{HF}$ und auf einem anderen analogen oder digitalen Anzeigeinstrument 124 den Spitzenwert des Stromes $I_{HF}$ eines jeden Koagulationsvorganges an. Die entsprechenden Meßwerte können dem Strommonitor 25 entnommen werden. Der Momentanwert des Stromes $I_{HF}$, welcher aus der Spannung $U_a$ des Strommonitors 25 abgeleitet wird, wird direkt angezeigt. Der Spitzenwert des Stromes $I_{HF}$ eines Koagulationsvorganges wird mittels eines Spitzenwertdetektors, bestehend aus der Diode 126, dem Kondensator 127 und dem Spannungsfolger 121 solange gespeichert und vom Instrument 124 angezeigt, bis durch das Signal c ein erneuter Koagulationsvorgang gestartet wird. Das Signal c triggert einen Monoflop 122, welcher einen Impuls mit einer derartigen Dauer an die Basis des Transistors 123 liefert, daß der Kondensator 127 ausreichend entladen wird.

Der Stromindikator 33 informiert den Operateur sowohl darüber, ob ein Strom $I_{HF}$ fließt, wieviel Strom $I_{HF}$ fließt als auch wie hoch der Strom $I_{HF}$ maximal während eines jeden Koagulationsvorganges angestiegen war.

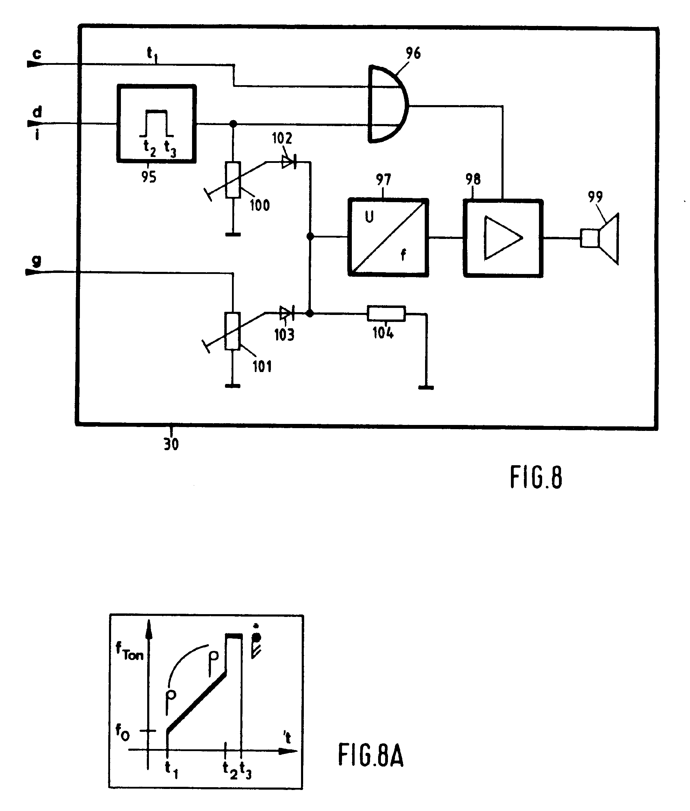

Anhand der Figuren 8 und 8a wird im folgenden ein Ausführungsbeispiel des akustischen Signalgebers 30 detaillierter beschrieben.

Dieser akustische Signalgeber 30 soll den Operateur auf akustischem Wege über den Koagulationsvorgang informieren. Deswegen erzeugt dieser akustische Signalgeber 30 variable Töne, welche dem jeweiligen Koagulationsvorgang zugeordnet sind. Im Zeitpunkt der Einschaltung des HF-Generators erzeugt dieser akustische Signalgeber 30 einen Ton mit der Frequenz $f_o$, beispielsweise 130 Hz wenn der Strom $I_{HF}$ Null ist. Der Spannungs/Frequenz-Wandler 97 ist so gestaltet, daß er bei $I_{HF} = g = $ Null, wenn also kein Strom $I_{HF}$ fließt, die Grundfrequenz $f_o$ erzeugt. Im Abschaltzeitpunkt $t_2$, der durch das Abschaltsignal d ausgelöst wird, springt die Frequenz des akustischen Signales auf eine fest eingestellte maximale Frequenz, beispielsweise 2000 Hz, welche staccato ertönt. Der staccato Ton $t_2$ bis $t_3$ kann entweder durch das Signal d oder durch das Signal i getriggert werden, je nachdem, wie der Schalter 32 geschaltet ist. Hierdurch hat der Operateur die einfache Möglichkeit, festzustellen, ob die automatische Abschaltung des Stromes $I_{HF}$ erst durch den Lichtbogenmonitor 26 erfolgt oder bereits, wie in der Regel zu erwarten, durch den Strommonitor 25. Der Grundton mit der Frequenz $f_0$ informiert den Operateur, daß der HF-Generator eingeschaltet ist. Aus der Tonfrequenz während des Zeitintervalles $t_1$ bis $t_2$ erkennt der Operateur die Intensität bzw. die Änderungen der Intensiät des Stromes $I_{HF}$. Aus dem hohen staccato Ton erkennt der Operateur das Ende des Koagulationsvorganges.

Die Tonfrequenz $f_{Ton}$ wird in einem Spannungs-Frequenz-Wandler 97, auch VCO genannt, erzeugt, in einem Verstärker 98 verstärkt und einem Lautsprecher 99 zugeleitet. Der Verstärker 98 wird über ein ODER-Glied 96 entweder durch das Einschaltsignal c oder für ein kurzes Zeitintervall $t_2$ bis $t_3$, staccato, durch das Signal d aktiviert und erzeugt entweder den im Spannungs/Frequenz-Wandler 97 voreingestellten Grundton $f_0$, wenn er durch das Signal c aktiviert wird oder den am Spannungsteiler 100 fest eingestellten staccato Ton mit der hohen Frequen von beispielsweise 2000 Hz, wenn er durch das Signal d aktiviert wird.

Fließt ein Strom $I_{HF}$, so wird die Tonfrequenz durch das Signal g, welches eine dem Strom $I_{HF}$ proportionale Spannung darstellt, wozu beispielsweise die Spannung $U_a$ aus dem Strommonitor 25 verwendet werden kann, proportional dieser Spannung in der Frequenz moduliert. Die Tonfrequenz steigt und fällt mit der Intensität des Stromes $I_{HF}$.

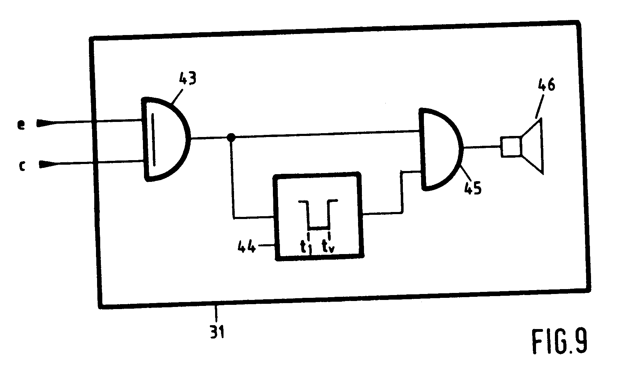

Anhand der Figur 9 wird im folgenden ein Ausführungsbeispiel des akustischen Signalgebers 31 detaillierter beschrieben. Dieser akustische Signalgeber erzeugt ein Warnsignal, wenn infolge eines Fehlers der HF-Oszillator 1 unbeabsichtigt eingeschaltet ist oder der HF-Oszillator nicht aktiviert wird, obwohl die Schaltvorrichtungen 21 und/oder 22 eingeschaltet haben. Hierzu werden die Signale c und e in einem EXOR-Glied verglichen, wobei folgende vier Möglichkeiten gegeben sein können.

| Fall | c | e | Ton | Bemerkungen |
|---|---|---|---|---|
| 1 | 0 | 0 | aus | Bestimmungsgemäße Funktion erfüllt |
| 2 | 0 | 1 | ein | HF-Oszillator ein, obwohl keine Schaltvorrichtung eingeschaltet ist. Es liegt ein Fehler vor! |
| 3 | 1 | 0 | ein | HF-Oszillator aus, obwohl Schaltvorrichtung 21 und/oder 22 eingeschaltet. Es liegt ein Fehler vor! |
| 4 | 1 | 1 | aus | HF-Oszillator ein und Schaltvorrichtung 21 und/oder 22 eingeschaltet. Bestimmungsgemäße Funktion erfüllt. |

Für den Fall 3 ist eine Verzögerung $t_1$ bis $t_v$ des Warnsignales (Ton) mittels eines Monoflops 44 vorhanden. Diese Verzögerung des Warnsignales verhindert, daß infolge unvermeidlicher, bauelemente- oder schaltungsbedingter Verzögerungen zwischen den Signalen c und e das Warnsignal auch bei jedem fehlerfreien Einschaltvorgang kurz ertönt.

Statt des Signales e kann diesem Signalgeber auch das Signal g zugeführt werden. Hierdurch wird insbesondere der Fall 3 in obiger Tabelle insofern vorteilhafter überwacht, als hierdurch auch Ausfälle der Steuerungseinrichtung 28, des HF-Oszillators 1, des Modulators 2, des Leistungsverstärkers 3, des Ausgangstransformators 4 und der Kondensatoren 5 und 6 überwacht werden. Hierbei muß die Verzögerung $t_1$ bis $t_v$ des Monoflops 44 so groß gewählt werden, daß während der Zeit, bis das Signal g einen ausreichenden Pegel für den Eingang des EXOR-Gliedes erreicht hat, kein Warnsignal erzeugt wird.

In einer weiteren Ausgestaltung dieses Signalgebers könnte die Verzögerung $t_1$ bis $t_v$ mit der Steuerungseinrichtung 28 so verknüpft werden, daß diese Verzögerung automatisch umgekehrt proportional zum Minimum-Pegel n und zur Anstiegsgeschwindigkeit v variiert wird.

Anhand der Figur 10 wird im folgenden ein Ausführungsbeispiel einer automatischen Schaltvorrichtung 22 detailliert beschrieben.

Automatische Schaltvorrichtungen bzw. Einschaltvorrichtungen sind bereits aus der DOS 2823291, DE 2540968 C2, DAS 1099658 und US 2,827,056 bekannt. Diese bekannten automatischen Einschaltvorrichtungen sind allerdings nicht direkt für die Anwendung in der erfindungsgemäßen Einrichtung entsprechend Figur 5 geeignet. Bei der Kombination dieser bekannten Einschaltvorrichtungen mit den erfindungsgemäßen automatischen Abschaltvorrichtungen 25 oder 26 entsprechend Figur 5 würden diese Einschaltvorrichtungen den HF-Generator nach der automatischen Abschaltung sofort wieder einschalten, wenn die Koagulationselektrode 13 oder 15 mit dem Gewebe des Patienten 17 in elektrisch leitfähigem Kontakt bleibt, was bei dieser Operationstechnik die Regel ist.

Die in Figur 10 dargestellte automatische Schaltvorrichtung 22 ist im Vergleich mit bekannten Schaltvorrichtungen insofern vorteilhaft weiterentwickelt, als eine Pause mit einstellbarer Dauer nach jeder automatischen Abschaltung eingefügt ist, während welcher der Operateur die Koagulationselektrode ohne Eile vom Gewebe abheben kann, bevor die automatische Schaltvorrichtung 22 den HF-Generator erneut einschaltet.

Als Kriterium für die automatische EIN/AUS-Schaltung des HF-Generators wird ein Kontrollstrom $I_k$ genutzt, welcher über die monopolare 15 oder bipolare Elektrode 13 durch das Gewebe des Patienten 17 fließt, wenn die monopolare Koagulationselektrode 15 und die Neutralelektrode 18 oder beide Pole der bipolaren Elektrode 13 gleichzeitig das Gewebe des Patienten 17 elektrisch leitfähig berühren. Hierfür enthält die automatische Schaltvorrichtung 22 eine Spannungsquelle $U_B$, welche vorzugsweise eine Wechselspannungsquelle ist und einen Stromindikator, welcher feststellt, ob ein Kontrollstrom $I_k$ fließt oder nicht. Als Spannungsquelle für die Spannung $U_B$ kann beispielsweise eine Wechselspannung mit 50 Hz aus einem Netztransformator dienen. Als Stromindikator kann beispielsweise ein Spannungskomparator 147 dienen, welcher den Spannungsabfall an einem Widerstand im Kontrollstromkreis überwacht, wobei Wechselspannung vorher gleichgerichtet werden muß. Um zu verhindern, daß der Hochfrequenz-Generator sofort eingeschaltet wird, wenn der Kontrollstrom $I_k$ fließt, ist eine nachtriggerbare Einschaltverzögerung vorhanden, welche aus den Elementen 131 bis 143 realisiert ist.

Berührt beispielsweise die bipolare Koagulationselektrode 13 mit beiden Polen gleichzeitig elektrisch leitfähig das Gewebe des Patienten 17, so wird die Basisspannung des Transistors 139 so klein, daß dieser Transistor sperrt. Hierdurch kann der Kondensator 142 über die Widerstände 140 und 143 mehr oder weniger schnell aufgeladen werden, je nachdem wie der Widerstand 143 eingestellt ist. Überschreitet die Spannung am positiven Eingang des Spannungskomparators 147 die am Spannungsteiler 144, 146 eingestellte Spannung, so wird der Ausgang b des Spannungskomparators 147 positiv und schaltet den HF-Generator ein. Die Einschaltverzögerung, das meint die Zeit zwischen elektrisch leitfähigem Berühren des Gewebes mit beiden Polen der bipolaren Koagulationselektrode 13 bis zu dem Zeitpunkt, in dem das Ausgangssignal b des Spannungskomparators 147 von negativ auf positiv kippt, kann entweder durch Änderung der Ladezeitkonstante mittels des Trimmwiderstandes 143 und/ oder durch Änderung der Spannung am negativen Eingang des Spannungskomparators 147 mittels des Trimmwiderstandes 146 eingestellt werden. Wird der elektrisch leitfähige Kontakt der bipolaren Koagulationselektrode 13 zum Gewebe unterbrochen, so wird der Transistor 139 sofort leitend und entlädt den Kondensator 142 sehr schnell über den Trimmwiderstand 141. Parallel zu dem Transistor 139 der bis hierher vorbekannten Schaltvorrichtung ist erfindungsgemäß ein zweiter Transistor 148 parallel geschaltet, welcher durch das Abschaltsignal d über die Leitung D mittels einer monostabilen Kippstufe 150 mit einstellbarer Impulsdauer leitfähig geschaltet wird und eine erneute Aufladung des Kondensators 142 erst nach Ablauf des eingestellten Impulses der monostabilen Kippstufe 150 zuläßt. Werden genauere Verzögerungen der Einschaltung und/oder Pause gewünscht, so kann diese Schaltvorrichtung 22 auch in digitaler Technik mit definiertem Impulsgenerator und Zählern sowie digitalen Komparatoren realisiert werden.

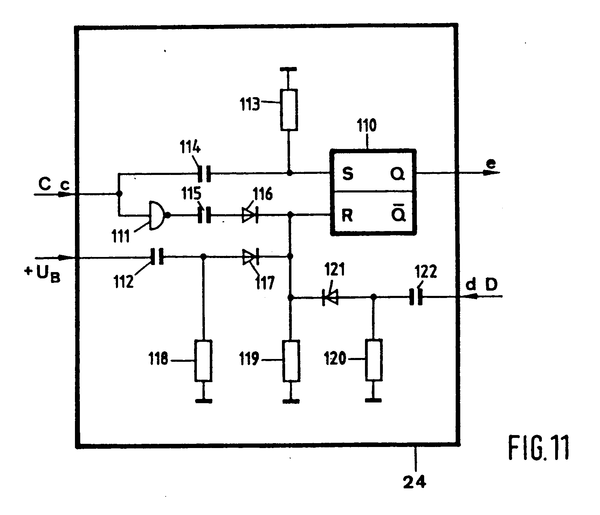

Anhand der Figur 11 wird ein Ausführungsbeispiel der Schaltlogik 24 detaillierter beschrieben.

Die Schaltlogik 24 soll die Ein- und Ausschaltung des HF-Oszillators 1 so koordinieren, daß nur definierte Schaltzustände möglich sind. Durch das Einschalten der Betriebsspannung $+U_B$ der Einrichtung wird eine bistabile Kippstufe 110, beispielsweise ein RS-Flip-Flop, am Reseteingang R so gesetzt, daß das Ausgangssignal e logisch Low-Pegel annimmt. Wird die Einrichtung durch die manuelle Schaltvorrichtung 21 und/oder durch die automatische Schaltvorrichtung 22 eingeschaltet, so wird hierbei das Signal c über die Leitung C auf logisch High-Pegel geschaltet, wodurch die bistabile Kippstufe 110 über den Eingang S so gesetzt wird, daß das Signal e logisch High-Pegel wird. Diesen Zustand behält die bistabile Kippstufe 110 solange, bis entweder das Signal d über die Leitung D auf High-Pegel oder das Signal c auf Low-Pegel kippt. Das Signal d kommt von den automatischen Abschalteinrichtungen 25, 26 oder 27 und wird über den Kondensator 122 direkt auf den Eingang R der bistabilen Kippstufe geführt. Das Signal c kommt von den Schaltvorrichtungen 21 und/oder 22 und muß bevor es auf den Eingang R der bistabilen Kippstufe 110 geführt wird in einem NICHT-Glied 111 invertiert werden.

Durch die dynamische Ansteuerung der bistabilen Kippstufe 110 über die Kondensatoren 112, 114, 115 und 122 wird verhindert, daß der HF-Generator nach dessen automatischer Abschaltung durch die automatischen Abschaltvorrichtungen 25, 26 oder 27 sofort wieder eingeschaltet wird, wenn das Signal c weiterhin High-Pegel führt. Nach jeder automatischen Aschaltung des HF-Generators muß auf diese Weise die Schaltvorrichtung 21 und/oder 22 erneut aktiviert werden.

Anhand der schematischen Figuren 12 und 13 wird im folgenden ein Ausführungsbeispiel eines Lichtbogen-Monitors 26 beschrieben.

Wie bereits weiter oben anhand der Figur 1 beschrieben, entstehen ab einem bestimmten Stadium des Koagulationsvorganges, nämlich dann, wenn infolge Koagulation und/oder Desikkation bzw. Austrocknung des biologischen Gewebes und/oder Dampfbildung im Grenzbereich zwischen der Kontaktfläche der Koagulationselektrode zum biologischen Gewebe eine elektrisch isolierende Schicht, innerhalb welcher bei ausreichend hoher Spannung $U_{HF}$ eine derart hohe elektrische Feldstärke entsteht, daß elektrische Lichtbogen zünden. Es ist bekannt, daß elektrische Lichtbogen nichtlineare elektrische Widerstände sind, durch welche der elektrische Strom der elektrischen Spannung nicht proportional ist. Es ist weiterhin bekannt, daß infolge der nichtlinearen Eigenschaft der elektrischen Lichtbogen, Wechselstrom, welcher durch elektrische Lichtbogen fließt gegenüber der treibenden Spannung derart verzerrt wird, daß hierdurch harmonische Frequenzen der Frequenz der Wechselspannung entstehen. Neu ist die Erkenntnis, daß infolge der stochastischen Eigenschaften der elektrischen Lichtbogen zwischen der Koagulationselektrode und biologischem Gewebe auch nichtharmonische Frequenzen der Frequenz der treibenden Spannung entstehen.

Solange zwischen der Koagulationselektrode 13, 15 und dem Gewebe kein elektrischer Lichtbogen vorhanden ist, sind am Ausgang des Hochfrequenzgenerators 11, 12 nur die Grundfrequenz $f_l$ sowie mehr oder weniger intensiv die ganzzahligen harmonischen Frequenzen $f_h$ der Grundfrequenz vorhanden. Die nullte harmonische Frequenz $f_o$ ist hierbei nicht vorhanden.

Sobald jedoch zwischen der Koagulationselektrode 13 oder 15 und dem Gewebe des Patienten 17 elektrische Lichtbogen zünden, entstehen infolge des nichtlinearen Verhaltens des elektrischen Widerstandes der Lichtbogen und infolge der stochastischen Häufigkeit der Lichtbogen pro Zeiteinheit auch nichtharmonische Frequenzen $f_n$ zwischen den harmonischen Frequenzen $f_h$.

Als Kriterium für die automatische Abschaltung des Stromes $I_{HF}$ wird erfindungsgemäß die Entstehung nichtharmonischer Frequenzen $f_n$ der Grundfrequenz $f_l$ des HF-Oszillators I während eines jeden Koagulationsvorganges genützt. Der Lichtbogen-Monitor 26 ist deshalb mit einem Filter 160 ausgestattet, welches selektiv eine bestimmte nichtharmonische Frequenz $f_n$ oder ein mehr oder weniger breites Frequenzspektrum zwischen zwei benachbarten harmonischen Frequenzen $f_h$ oder mehrere mehr oder weniger breite Frequenzspektren der nichtharmonischen Frequenzen $f_n$ zwischen verschiedenen benachbarten harmonischen Frequenzen $f_h$ der Grundfrequenz $f_l$ des HF-Oszillators I durchläßt und sowohl die Grundfrequenz $f_l$ als auch die relevanten harmonischen Frequenzen $f_h$ des HF-Oszillators I ausreichend stark dämpft.

Das Ausgangssignal k des Filters 160 wird einem Spannungskomparator 162 zugeführt dessen Ausgangssignal r eine bistabile Kippstufe 161 so beeinflußt, daß sie ein Abschaltsignal d auf die Leitung D liefert, wodurch der Strom $I_{HF}$ abgeschaltet wird bis durch erneutes Einschalten über manuelle 21 und/oder automatische 22 Schaltvorrichtungen über eine Leitung C ein Einschaltsignal c die bistabile Kippstufe 161 wieder so beeinflußt, daß der Strom $I_{HF}$ wieder eingeschaltet wird. Die Schwellenspannung des Spannungskomparators 162 kann an einer Spannungsquelle 163 eingestellt werden.

Das Filter 160 kann in verschiedenen Technologien gebaut werden. Geeignet ist beispielsweise ein Keramikfilter, welches eine oder mehrere der nichtharmonischen Frequenzen $f_n$ unterhalb der Grundfrequenz $f_l$ des HF-Oszillators durchläßt und sowohl die Grundfrequenz $f_l$ als auch deren Harmonischen inklusive die Frequenz Null dämpft. Keramikfilter haben den Vorteil, daß sie relativ preiswert hergestellt werden können, eine ausreichende elektrische Isolation zwischen Filtereingang und Filterausgang bieten und daß mehrere derartige Filter, welche auf verschiedene diskrete nichtharmonische Frequenzen und/oder Frequenzbänder zwischen zwei benachbarten harmonischen Frequenzen abgestimmt sind, preiswert parallelgeschaltet werden können, wodurch die Redundanz des Lichtbogenmonitors gesteigert wird.

In der Figur 13 ist eine idealisierte Filtercharakteristik eines für den Lichtbogen-Monitor 26 geeigneten Filters 160 dargestellt. $U_e$ meint die Eingangsspannung, $U_a$ die Ausgangsspannung des Filters. Die Frequenz $f_h$ ist eine relevante harmonische Frequenz der Grundfrequenz $f_l$ inklusive dieser Grundfrequenz selbst. Relevant sind nur die harmonischen Frequenzen, welche im Frequenzspektrum des Hochfrequenzgenerators vorhanden sind. Die Frequenz $f_h$, ist eine beliebige harmonische Frequenz und $f_{h+l}$ meint die nächst höhere relevante harmonische Frequenz. Sowohl die untere Grenzfrequenz $f_u$ als auch die obere Grenzfrequenz $f_o$ des Filters 160 müssen so bemessen sein, daß ausreichende Abstände zu den harmonischen Frequenzen $f_h$ und $f_{h+l}$ vorhanden sind, so daß Toleranzen der Frequenz $f_l$ und damit derer Harmonischen berücksichtigt sind.

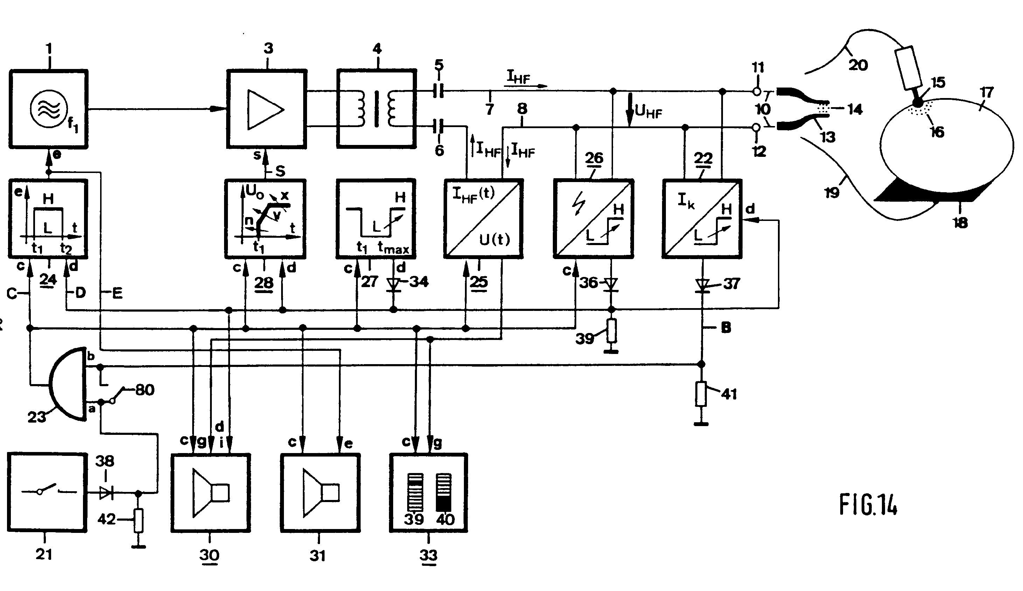

Anhand der Figur 14 wird im folgenden ein Ausführungsbeispiel einer Einrichtung zur thermischen Koagulation biologischen Gewebes mittels hochfrequenten elektrischen Wechselstromes beschrieben, welche die Aufgabe der Erfindung auf andere Weise als das anhand der Figuren 4 und 5 beschriebene Ausführungsbeispiel löst. In dieser Lösung erfolgt die Abschaltung des Stromes $I_{HF}$ primär durch den Lichtbogen-Monitor 26, welcher anhand der Figuren 12 und 13 detaillierter beschrieben wird. Bei dieser Lösung dient der Strom-Monitor 25 lediglich zur Umwandlung der Amplitude des Stromes $I_{HF}$ (t) in eine diesem Strom proportionale Spannung U(t), wofür beispielsweise die Elemente 50, 51, 52, 53, 55, 58, 59, 60 und 62 in der in Figur 2 dargestellten Anordnung verwendet werden können. Alle weiteren vorteilhaften Weiterentwicklungen dieses Ausführungsbeispieles sind identisch mit den in den Figuren 5 bis 11 dargestellten und beschriebenen Weiterentwicklungen der in Figur 4 dargestellten Lösung.

**Patentansprüche**

1.  Hochfrequenz-Chirurgiegerät für die thermische Koagulation biologischer Gewebe mittels hochfrequenten elektrischen Wechselstromes, mit einem Hochfrequenzgenerator, mit Einschalteinrichtungen, über welche der HF-Strom manuell oder automatisch aktiviert werden kann, sowie mit einer Abschalteinrichtung zur automatischen Beendigung eines Koagulationsvorganges in Abhängigkeit von Änderungen der elektrischen Leitfähigkeit des Koagulates, **dadurch gekennzeichnet,** daß ein Lichtbogen-Monitor (26) zur Überwachung der Entstehung nichtharmonischer Frequenzen der Grundfrequenz ($f_l$) des Hochfrequenzgenerators (I) an den Ausgang (11,12) des Chirurgiegeräts angeschlossen ist, der ein Filter (160) enthält, welches mindestens eine der von den elektrischen Lichtbogen zwischen der Koagulationselektrode (15;13) und dem Gewebe des Patienten erzeugten nichtharmonischen Frequenzen der Grundfrequenz ($f_l$) des Hochfrequenzgenerators (I) aus dem HF-Strom ($I_{HF}$) durchläßt und gleichzeitig sowohl die Grundfrequenz ($f_l$) als auch deren harnomische Frequenzen dämpft, und daß das Ausgangssignal (K) des Filters (160) einem Spannungskomparator (162) zugeleitet wird, dessen Ausgangssignal (r) eine bistabile Kippstufe (161) so zurücksetzt, daß deren Ausgangssignal (Q) den HF-Strom ($I_{HF}$) abschaltet, bis durch ein bei Betätigen einer manuellen Schaltvorrichtung (21) und/oder durch eine automatische Schaltvorrichtung (22) erzeugtes Signal (C), das der Kippstufe (161) zugeführt wird, der HF-Strom ($I_{HF}$) wieder eingeschaltet wird.

2.  Hochfrequenz-Chirurgiegerät nach Anspruch 1, **dadurch gekennzeichnet,** daß zur Überwachung der Amplitudenschwankungen des HF-Stromes ($I_{HF}$) und/oder der entsprechenden Wechselspannung ($U_{HF}$) während jedes Koagulationsvorganges ein Strommonitor (25) vorgesehen ist, der mittels eines Strom/Spannungswandlers (50 bis 52) eine den Amplitudenschwankungen (A(t)) des HF-Stromes ($I_{HF}$) proportionale elektrische Spannung $U = f(I_{HF})$ erzeugt, aus welcher mittels eines ersten Detektors (53,55,58,60,62) eine den Amplitudenschwankungen (A(t)) des HF-Stromes ($I_{HF}$) proportionale erste Gleichspannung ($U_a$) und mittels eines zweiten Detektors (54,56,61,63), der als Spitzenwertdetektor wirkt, eine zweite Gleichspannung ($U_b$) gebildet wird, die proportional mit der Amplitude (A(t)) des HF-Stromes ($I_{HF}$) ansteigt, daß die erste Gleichspannung ($U_a$) und die mittels eines Spannungsteilers (63) um einen einstellbaren Faktor (k) heruntergeteilte zweite Gleichspannung ($kU_b$) einem Spannungskomparator (67) zugeführt werden, dessen Ausgangssignal eine bistabile Kippstufe (70) setzt, sobald die erste Gleichspannung ($U_a$) kleiner wird als die heruntergeteilte zweite Gleichspannung ($kU_b$), und daß das Ausgangssignal (Q) der Kippstufe (70) den HF-Strom ($I_{HF}$) abschaltet, bis durch Betätigen einer manuellen Schaltvorrichtung (21) und/oder durch eine automatische Schaltvorrichtung (22) der HF-Strom ($I_{HF}$) wieder eingeschaltet wird.

3.  Hochfrequenz-Chirurgiegerät nach Anspruch 1 oder 2, **dadurch gekennzeichnet,** daß in dem Chirurgiegerät sowohl ein Strommonitor (25) mit der zugeordneten Einrichtung nach Anspruch 1 als auch ein Lichtbogen-Monitor (26) mit der zugeordneten Einrichtung nach Anspruch 2 vorgesehen sind.

4.  Hochfrequenz-Chirurgiegerät nach einem der vorhergehenden Ansprüche, **dadurch gekennzeichnet,** daß die Leerlaufspannung ($U_o$) des Hochfrequenzgenerators durch eine Steuerungseinrichtung (28) ab dem Zeitpunkt ($t_1$) einer jeden Einschaltung des Hochfrequenzgenerators von einem einstellbaren Minimum-Pegel ($U_{min}$) mit einstellbarer Geschwindigkeit (v) so lange ansteigt, bis der Hochfrequenzgenerator durch den Strommonitor (25) oder den Lichtbogen-Monitor (26) oder durch einen Einschaltdauer-Begrenzer (27) automatisch oder durch die manuelle Schaltvorrichtung (21) manuell abgeschaltet wird, und daß ein Maximum pegel ($U_{max}$) der Spannung des Hochfrequenzgenerators einstellbar ist, welcher so lange erhalten bleibt, bis der Hochfrequenzgenerator durch eine der genannten Abschaltvorrichtungen (25,26,27 oder 21) abgeschaltet wird, wobei gleichzeitig die Spannung ($U_{HF}$) des Hochfrequenzgenerators auf den Pegel Null zurückgeschaltet wird.

5.  Hochfrequenz-Chirurgiegerät nach Anspruch 4, **dadurch gekennzeichnet,** daß die Steuerungseinrichtung (28) mittels eines Digital/Analog-Wandlers (83) ein analoges Steuerungssignal (S) erzeugt, dessen Pegel digital mittels eines programmierbaren Zählers (82) gebildet wird, wobei der Minimum-Pegel (n) an einem digitalen Vorwahlschalter (81) einstellbar ist und im Zeitpunkt ($t_1$) der Einschaltung des Hochfrequenzgenerators in den Zähler (82) übernommen wird, wenn der Hochfrequenzgenerator eingeschaltet wird, daß im Einschaltzeitpunkt ($t_1$) ein Impulsgenerator (86) mit einstellbarer Impulsfrequenz (v) gestartet wird, dessen Impulse dem Zähler (82) zugeführt werden und von diesem, ausgehend von der Minimum-Zahl (n) aufaddiert werden, daß am Ausgang des Zählers (82) sowohl ein Digital/Analog-Wandler (83), welcher die vom Zähler gelieferte digitale Zahl um einen analogen Spannungspegel (s) wandelt, als auch ein Digital-Komparator (84) angeschlossen ist, welcher die vom Zähler (82) gelieferte digitale Zahl mit einer an einem digitalen Vorwahlschalter (85), dem Maximum-Pegel entsprechenden digitalen Zahl (x) vergleicht und bei Gleichstand der beiden digitalen Zahlen den Impulsgenerator (86) stoppt, so daß ab diesem Zeitpunkt das Steuersignal (S) konstant bleibt, und daß die Steuerungseinrichtung (28) einen Stopp-Eingang hat, welcher den Zähler (82) über dessen Reseteingang (RS) und damit auch das Steuerungssignal (S) auf Null zurücksetzt, sobald ein Abschaltsignal (d) vorhanden ist.

6. Hochfrequenz-Chirurgiegerät nach einem der vorhergehenden Ansprüche, **dadurch gekennzeichnet,** daß eine automatische Schaltvorrichtung vorgesehen ist, welche den Hochfrequenzgenerator sofort oder nach einer einstellbaren und retriggerbaren Verzögerungsdauer automatisch einschaltet, wenn eine monopolare Koagulationselektrode (15) und eine Neutralelektrode (18) oder beide Pole einer bipolaren Koagulationselektrode (13) gleichzeitig elektrisch leitfähig das Gewebe des Patienten berühren, und daß die automatische Schaltvorrichtung eine monostabile Kippstufe (150) enthält, welche durch ein Abschaltsignal (d) getriggert wird und eine erneute automatische Einschaltung des Hochfrequenzgenerators erst dann zuläßt, wenn die an der monostabilen Kippstufe (150) einstellbare Impulsdauer beendet ist.

7. Hochfrequenz-Chirurgiegerät nach einem der vorhergehenden Ansprüche, **dadurch gekennzeichnet,** daß ein Stromindikator (33) vorgesehen ist, welcher auf einem ersten analogen oder digitalen Anzeigeinstrument (125) den Momentanwert des hochfrequenten Wechselstromes ($I_{HF}$) und auf einem zweiten analogen oder digitalen Anzeigeinstrument (124) den Spitzenwert des hochfrequenten Wechselstromes ($I_{HF}$) eines jeden Koagulationsvorganges anzeigt, wobei der Spitzenwert auch nach dem Abschalten des Hochfrequenzgenerators bis zu dem Zeitpunkt angezeigt bleibt, in welchem der Hochfrequenzgenerator erneut eingeschaltet wird, wobei ein Spitzenwertspeicher (127) mittels einer Entladeeinrichtung (122,123,128) entladen wird und sofort den neuen Spitzenwert übernimmt.

8. Hochfrequenz-Chirurgiegerät nach einem der vorhergehenden Ansprüche, **dadurch gekennzeichnet,** daß ein akustischer Signalgeber (30) vorhanden ist, welcher in Abhängigkeit von den verschiedenen Stadien eines Koagulationsvorganges unterschiedliche Töne erzeugt, wobei ein Grundton mit einer Frequenz ($f_o$) von beispielsweise 130 Hz ertönt, wenn der Hochfrequenzgenerator eingeschaltet ist, jedoch kein hochfrequenter Wechselstrom ($I_{HF}$) fließt, die Tonfrequenz proportional zur Intensität des Stromes ($I_{HF}$) ansteigt oder fällt, und ein staccato Ton mit hoher Tonfrequenz, vorzugsweise 2000 Hz, ab dem Zeitpunkt ($t_2$ bis $t_3$) ertönt, wenn der Hochfrequenzgenerator abgeschaltet wird.

9. Hochfrequenz-Chirurgiegerät nach einem der vorhergehenden Ansprüche, **dadurch gekennzeichnet,** daß ein akustischer Signalgeber (31) vorhanden ist, welcher ein Warnsignal abgibt, wenn der Hochfrequenzgenerator (1) eingeschaltet ist, obwohl keine Schaltvorrichtung (21 oder 22) eingeschaltet ist oder wenn der Hochfrequenzgenerator (1) ausgeschaltet ist, obwohl eine Schaltvorrichtung (21 und/oder 22) eingeschaltet ist, wobei dieses Signal durch eine monostabile Kippstufe (44) zeitlich gegenüber dem Zeitpunkt ($t_1$) jeder Einschaltung des Hochfrequenzgenerators verzögert ertönt.

10. Hochfrequenz-Chirurgiegerät nach einem der Ansprüche 1 oder 3 bis 9, **dadurch gekennzeichnet,** daß der Hochfrequenzgenerator (1) über eine UND-Schaltung (23) nur dann einschaltbar ist, wenn sowohl die automatische Schaltvorrichtung (22) als auch eine manuelle Schaltvorrichtung (21) gleichzeitig Einschaltsignale (b,a) an die UND-Schaltung (23) liefern.

11. Hochfrequenz-Chirurgiegerät nach einem der vorhergehenden Ansprüche, **dadurch gekennzeichnet,** daß ein Einschaltdauer-Begrenzer (27) zur automatischen Abschaltung des Hochfrequenzgenerators nach einer voreinstellbaren Zeitspanne vorgesehen ist, und daß die maximale Einschaltdauer ($t_1$ bis $t_{max}$) durch das Einschaltsignal (C) nachgetriggert wird.

## Claims

1. A high-frequency surgical apparatus for the thermal coagulation of biological tissues by means of high-frequency electrical alternating current, with a high-frequency generator, with starting devices, via which the high-frequency current can be activated manually or automatically, and also with a stopping device for the automatic termination of a coagulation process as a function of changes to the electrical conductivity of the coagulate, characterised in that an arc monitor (26) for monitoring the formation of non-har-

13

monic frequencies of the fundamental frequency ($f_1$) of the high-frequency generator (1) is connected to the outlet (11,12) of the surgical apparatus, which contains a filter (160), which allows through at least one of the nonharmonic frequencies of the fundamental frequency ($f_1$) of the high frequency generator (1) from the high-frequency current ($I_{HF}$) produced from the electric arcs between the coagulation electrode (15;13) and the patient's tissue, and at the same time damps both the fundamental frequency ($f_1$) and also its harmonic frequencies, and that the output signal (K) of the filter (160) is supplied to a voltage comparator (162), the output signal (r) of which resets a bistable flip-flop (161) such that its output signal (Q) switches off the high-frequency current ($I_{HF}$), until the high-frequency current ($I_{HF}$) is switched on again by a signal (C), produced on actuation of a manual switching device (21) and/or by an automatic switching device (22), which signal is passed to the flip-flop (161).

2.  A high-frequency surgical apparatus according to Claim 1, characterised in that for monitoring the amplitude fluctuations of the high-frequency current ($I_{HF}$) and/or of the corresponding alternating current ($U_{HF}$) during each coagulation process a current monitor (25) is provided, which by means of a current/voltage converter (50 to 52) produces an electrical voltage U = f $_{(HF)}$ proportional to the amplitude fluctuations (A(t)) of the high-frequency current ($I_{HF}$), from which, by means of a first detector (53,55,58,60,62), a first direct voltage ($U_a$) is formed, proportion to the amplitude fluctuations (A(t)) of the high-frequency current ($I_{HF}$) and by means of a second detector (54,56,61,63), which acts as peak value detector, a second direct voltage ($U_b$) is formed, which rises in proportion with the amplitude (A(t)) of the high-frequency current ($I_{HF}$), that the first direct voltage ($U_a$) and the second direct voltage ($kU_b$), subdivided by means of a voltage divider (63) by an adjustable factor (k) are supplied to a voltage comparator (67), the output signal of which sets a bistable flip-flop (70), as soon as the first direct voltage ($U_a$) becomes smaller than the subdivided second direct voltage ($kU_b$), and that the output signal (Q) of the flip-flop (70) switches off the high-frequency current ($I_{HF}$), until through actuation of a manual switching device (21) and/or through an automatic switching device (22) the high-frequency current ($I_{HF}$) is switched on again.

3.  A high-frequency surgical apparatus according to Claim 1 or 2, characterised in that in the surgical apparatus there are provided both a current monitor (25) with the associated arrangement according to Claim 1, and also an arc monitor (26) with the associated arrangement according to Claim 2.

4.  A high-frequency surgical apparatus according to one of the preceding claims, characterised in that the open-circuit voltage ($U_o$) of the high-frequency generator rises through a control arrangement (28) from the instant ($t_1$) of each switching on of the high-frequency generator from an adjustable minimum level ($U_{min}$) with adjustable speed (v), until the high-frequency generator is switched off by the current monitor (25) or the arc monitor (26) or by a connection duration limiter (27) automatically or manually by the manual switching device (21), and that a maximum level ($U_{max}$) of the voltage of the high frequency generator is adjustable, which is maintained until the high-frequency generator is switched off by one of the said stopping devices (25,26,27 or 21), in which at the same time the voltage ($U_{HF}$) of the high-frequency generator is switched back to the zero level.

5.  A high-frequency surgical apparatus according to Claim 4, characterised in that the control arrangement (28) produces an analog control signal (S) by means of a digital/analog converter (83), the level of which signal is formed digitally by means of a programmable counter (82), in which the minimum level (n) is adjustable on a digital preselection switch (81) and at the instant ($t_1$) of switching on the high-frequency generator is taken over into the counter (82), when the high-frequency generator is switched on, that at the instant of switching on ($t_1$) a pulse generator (86) with adjustable pulse frequency (v) is started, the pulses of which are supplied to the counter (82) and are added up by this, starting from the minimum number (n), that at the output of the counter (82) there are connected both a digital/analog converter (83), which converts the digital number delivered from the counter by an analog voltage level(s), and also a digital comparator (84), which compares the digital signal delivered from the counter (82) with a digital number (x) on a digital preselection switch (85) corresponding to the maximum level, and stops the pulse generator (86) when the two digital numbers are identical, so that from this instant the control signal (S) remains constant, and that the control arrangement (28) has a stop input, which resets the counter (82) via its reset input (RS) and hence also the control signal (S) to zero, as soon as a switch-off signal (d) is present.

6.  A high-frequency surgical apparatus according to one of the preceding claims, characterised in that an automatic switching device is provided, which automatically switches on the high-frequency generator immediately or after an adjustable and retriggerable delay period, when a monopolar coagulation electrode

14

(15) and a neutral electrode (18) or both poles of a bipolar coagulation electrode (13), simultaneously touch the tissue of the patient in an electrically conductive manner, and that the automatic switching device contains a monostable flip-flop (150), which is triggered by a switch-off signal (d) and only permits a renewed automatic switching on of the high-frequency generator when the pulse duration adjustable on the monostable flip-flop (150) is terminated.

7. A high-frequency surgical apparatus according to one of the preceding claims, characterised in that a current indicator (33) is provided, which on a first analog or digital display instrument (125) indicates the instantaneous value of the high-frequency alternating current ($I_{HF}$) and on a second analog or digital display instrument (124) indicates the peak value of the high-frequency alternating current ($I_{HF}$) of each coagulation process, in which the peak value also remains indicated after switching off of the high-frequency generator up to the instant at which the high-frequency generator is switched on again, in which a peak value memory (127) is discharged by means of a discharge arrangement (122,123,128) and immediately takes over the new peak value.

8. A high-frequency surgical apparatus according to one of the preceding claims, characterised in that an acoustic signal emitter (30) is present, which produces different tones as a function of the various stages of a coagulation process, in which a basic tone sounds with a frequency ($f_o$) of for example 130 Hz, when the high-frequency generator is switched on, but no high-frequency alternating current ($I_{HF}$) flows, the tone frequency rises or falls in proportion to the intensity of the current ($I_{HF}$), and a staccato tone sounds with a high tone frequency, preferably 2000 Hz, from the instant ($t_2$ to $t_3$) when the high-frequency generator is switched off.

9. A high-frequency surgical apparatus according to one of the preceding claims, characterised in that an acoustic signal emitter (31) is present, which emits a warning signal when the high-frequency generator (1) is switched on, although no switching device (21 or 22) is switched on or when the high-frequency generator (2) is switched off, although a switching device (21 and/or 22) is switched on, in which this signal sounds in a chronologically delayed manner through a monostable flip-flop (44) with respect to the instant ($t_1$) of each switching on of the high-frequency generator.

10. A high-frequency surgical apparatus according to one of Claims 1 or 3 to 9, characterised in that the high-frequency generator (1) is only able to be switched on via an AND-circuit (23) when both the automatic switching device (22) and also a manual switching device (21) simultaneously deliver switching on signals (b,a) to the AND-circuit (23).

11. A high-frequency surgical apparatus according to one of the preceding claims, characterised in that a connection duration limiter (27) is provided to automatically switch off the high-frequency generator after a previously adjustable timespan, and that the maximum connection duration ($t_1$ to $t_{max}$) is subsequently triggered by the connection signal (C).

**Revendications**

1. Appareil chirurgical à haute fréquence pour la coagulation thermique de tissus biologiques au moyen d'un courant électrique alternatif de haute fréquence, comprenant un oscillateur haute fréquence, des dispositifs de connexion permettant d'activer manuellement ou automatiquement le courant H.F. ainsi qu'un dispositif de déconnexion pour mettre fin automatiquement à une opération de coagulation en fonction de variations de la conductivité du coagulat, **caractérisé** par le fait qu'un moniteur d'arc électrique (26) destiné à surveiller l'apparition de fréquences non-harmoniques de la fréquence de base ($f_1$) de l'oscillateur haute fréquence (1) est raccordé à la sortie (11, 12) de l'appareil chirurgical, qui contient un filtre (160) qui, dans le courant H.F. ($I_{HF}$), laisse passer au moins l'une des fréquences non-harmoniques de la fréquence de base ($f_1$) de l'oscillateur haute fréquence (1), produites par les arcs électriques entre l'électrode de coagulation (15; 13) et le tissu du patient, et atténue simultanément aussi bien la fréquence de base ($f_1$) que ses fréquences harmoniques et que le signal de sortie (K) du filtre (160) est transmis à un comparateur de tension (162) dont le signal de sortie (r) remet une bascule bistable (161) à l'état initial de telle manière que son signal de sortie (Q) coupe le courant H.F. ($I_{HF}$) jusqu'à ce que le courant H.F. ($I_{HF}$) soit de nouveau rétabli par un signal (C) qui est produit lors de l'actionnement d'un dispositif de commutation manuel (21) et/ou par un dispositif de commutation automatique (22) et est transmis à la bascule (161).

2. Appareil chirurgical à haute fréquence selon la revendication 1, **caractérisé** par le fait que pour surveiller les variations d'amplitude du courant H.F. ($I_{HF}$) et/ou de la tension alternative ($U_{HF}$) correspondante pendant chaque opération de coagulation, il est prévu un moniteur de courant (25) qui produit au moyen d'un convertisseur courant-tension (50 à 52) une tension électrique $U = f(I_{HF})$ qui est proportionnelle aux variations d'amplitude ($A(t)$) du courant H.F. ($I_{HF}$) et à partir de laquelle on forme au moyen d'un premier détecteur (53, 55, 58, 60, 62) une première tension continue ($U_a$) proportionnelle aux variations d'amplitude ($A(t)$) du courant H.F. ($I_{HF}$) et au moyen d'un deuxième détecteur (54, 56, 61, 63) qui fonctionne comme détecteur de valeur de crête, une deuxième tension continue ($U_b$) qui augmente proportionnellement avec l'amplitude ($A(t)$) du courant H.F. ($I_{HF}$), que la première tension continue ($U_a$) et la deuxième tension continue ($kU_b$) réduite par division d'un facteur ($k$) réglable au moyen d'un diviseur de tension (63) sont transmises à un comparateur de tension (67) dont le signal de sortie active une bascule bistable (70) dès que la première tension continue ($U_a$) devient inférieure à la deuxième tension continue ($kU_b$) réduite par division et que le signal de sortie ($Q$) de la bascule (70) coupe le courant H.F. ($I_{HF}$), jusqu'à ce que le courant H.F. ($I_{HF}$) soit de nouveau rétabli par l'actionnement d'un dispositif de commutation manuel (21) et/ou par un dispositif de commutation automatique (22).

3. Appareil chirurgical à haute fréquence selon la revendication 1 ou 2, **caractérisé** par le fait qu'aussi bien un moniteur de courant (25) avec le dispositif associé selon la revendication 1 qu'un moniteur d'arc électrique (26) avec le dispositif associé selon la revendication 2 sont prévus dans l'appareil chirurgical.

4. Appareil chirurgical à haute fréquence selon l'une des revendications précédentes, **caractérisé** par le fait qu'un dispositif de commande (28) fait augmenter la tension de marche à vide ($U_o$) de l'oscillateur haute fréquence, à partir du moment ($t_1$) de chaque mise en circuit de l'oscillateur haute fréquence, à partir d'un niveau minimum ($U_{min}$) réglable, avec une vitesse ($v$) réglable, jusqu'à ce que l'oscillateur haute fréquence soit mis hors circuit automatiquement par le moniteur de courant (25) ou le moniteur d'arc électrique (26) ou par un limiteur de durée de mise en circuit (27) ou manuellement par le dispositif de commutation manuel (21) et qu'il est possible de régler un niveau maximum ($U_{max}$) de la tension de l'oscillateur haute fréquence, lequel est maintenu jusqu'à ce que l'oscillateur haute fréquence soit mis hors circuit par l'un des dispositifs de déconnexion (25, 26, 27 ou 21) mentionnés, la tension ($U_{HF}$) de l'oscillateur haute fréquence étant de ce fait simultanément ramenée au niveau zéro.

5. Appareil chirurgical à haute fréquence selon la revendication 4, **caractérisé** par le fait que le dispositif de commande (28) produit au moyen d'un convertisseur numérique-analogique (83) un signal de commande analogique ($S$) dont le niveau est formé de manière numérique au moyen d'un compteur (82) programmable, le niveau minimum ($n$) pouvant être réglé sur un présélecteur (81) numérique et étant transféré dans le compteur (82) au moment ($t_1$) de la mise en circuit de l'oscillateur haute fréquence lorsque l'oscillateur haute fréquence est mis en circuit, qu'un générateur d'impulsions (86) à fréquence d'impulsions ($v$) réglable est mis en marche au moment de connexion ($t_1$), les impulsions dudit générateur étant transmises au compteur (82) et étant additionnées par ce dernier à partir du nombre minimum ($n$), qu'il est raccordé à la sortie du compteur (82) un convertisseur numérique-analogique (83) qui convertit le nombre numérique fourni par le compteur en un niveau de tension analogique ($s$) ainsi qu'un comparateur numérique (84) qui compare le nombre numérique fourni par le compteur (82) avec le nombre numérique ($x$) d'un présélecteur numérique (85) correspondant au niveau maximum et arrête le générateur d'impulsions (86) en cas d'égalité des deux nombres numériques si bien qu'à partir de ce moment le signal de commande ($S$) reste constant et que le dispositif de commande (28) possède une entrée d'arrêt qui ramène le compteur (82) à zéro par l'intermédiaire de son entrée de remise à zéro (RS) et partant, également le signal de commande ($S$) dès qu'un signal d'arrêt ($d$) existe.

6. Appareil chirurgical à haute fréquence selon l'une des revendications précédentes, **caractérisé** par le fait qu'il est prévu un dispositif de commutation automatique qui connecte automatiquement l'oscillateur haute fréquence immédiatement ou après un délai réglable et pouvant être redéclenché, lorsqu'une électrode de coagulation monopolaire (15) et une électrode neutre (18) ou les deux pôles d'une électrode de coagulation bipolaire (13) sont simultanément en contact électroconducteur avec le tissu du patient, et que le dispositif de commutation automatique contient une bascule monostable (150) qui est déclenchée par un signal d'arrêt ($d$) et n'autorise une nouvelle mise en circuit automatique de l'oscillateur haute fréquence que lorsque la durée d'impulsion réglable sur la bascule monostable (150) est écoulée.

7. Appareil chirurgical à haute fréquence selon l'une des revendications précédentes, **caractérisé** par le fait qu'il est prévu un indicateur de courant (33) qui indique sur un premier instrument d'affichage (125) analogique ou numérique la valeur momentanée du courant alternatif de haute fréquence ($I_{HF}$) et sur un deuxième instrument d'affichage (124) analogique ou numérique la valeur de crête du courant alternatif de haute fréquence ($I_{HF}$) de chaque opération de coagulation, la valeur de crête restant affichée même après la déconnexion de l'oscillateur haute fréquence jusqu'au moment où l'oscillateur haute fréquence est de nouveau mis en circuit, une mémoire de valeur de crête (127) étant déchargée au moyen d'un dispositif de décharge (122, 123, 128) et recevant aussitôt la nouvelle valeur de crête.

8. Appareil chirurgical à haute fréquence selon l'une des revendications précédentes, **caractérisé** par le fait qu'il existe un transmetteur de signal acoustique (30) qui produit des sons différents en fonction des différents stades d'une opération de coagulation, un son fondamental avec une fréquence ($f_o$) de par exemple 130 Hz retentissant lorsque l'oscillateur haute fréquence est connecté mais qu'aucun courant alternatif de haute fréquence ($I_{HF}$) ne passe, la fréquence du son augmentant ou diminuant proportionnellement à l'intensité du courant ($I_{HF}$) et un son staccato d'une fréquence plus élevée, de préférence de 2000 Hz, retentissant à partir du moment ($t_2$ à $t_3$) où l'oscillateur haute fréquence est déconnecté.

9. Appareil chirurgical à haute fréquence selon l'une des revendications précédentes, **caractérisé** par le fait qu'il existe un transmetteur de signal acoustique (31) qui délivre un signal d'avertissement lorsque l'oscillateur haute fréquence (1) est connecté bien qu'aucun dispositif de commutation (21 ou 22) ne soit connecté ou lorsque l'oscillateur haute fréquence (1) est déconnecté bien qu'un dispositif de commutation (21 et/ou 22) soit connecté, le retentissement de ce signal étant retardé par une bascule monostable (44) par rapport au moment ($t_1$) de chaque connexion de l'oscillateur haute fréquence.

10. Appareil chirurgical à haute fréquence selon l'une des revendications 1 ou 3 à 9, **caractérisé** par le fait que l'oscillateur haute fréquence (1) peut être connecté par l'intermédiaire d'un circuit ET uniquement lorsque le dispositif de commutation automatique (22) et le dispositif de commutation manuel (21) délivrent simultanément des signaux de connexion (b, a) au circuit ET (23).

11. Appareil chirurgical à haute fréquence selon l'une des revendications précédentes, **caractérisé** par le fait qu'un limiteur de durée de mise en circuit (27) est prévu pour la mise hors circuit automatique de l'oscillateur haute fréquence après une période de temps pouvant être réglée préalablement et que la durée maximale de mise en circuit ($t_1$ à $t_{max}$) est redéclenchée par le signal de mise en circuit (C).

FIG.1

EP 0 430 929 B1

FIG.2

FIG.3

EP 0 430 929 B1

FIG.4

FIG.4A

FIG.5

EP 0 430 929 B1

FIG.6

FIG.7

FIG.8

FIG.8A

FIG.9

FIG.10

EP 0 430 929 B1

FIG. 11

FIG.12

FIG.13

FIG.14